# EUROPEAN PATENT APPLICATION

(11) **EP 4 541 339 A1**
(43) Date of publication of application: **23.04.2025**
(21) Application number: 23882324.9
(22) Date of filing: 27.09.2023
(51) Int. Cl.: A61K 8/02, A61Q 1/00, A61Q 1/02, A61Q 1/06, A61Q 1/08, A61Q 1/10

(54) **SOLID PERSONAL CARE PRODUCT AND METHOD FOR PRODUCING SOLID PERSONAL CARE PRODUCT**

(30) Priority: 28.10.2022 JP 2022173803; 22.09.2023 JP 2023159278
(71) Applicant: Kao Corporation, Tokyo 103-8210 (JP)
(72) Inventor: KIBE, Yoshiyuki, Tokyo 103-8210 (JP); NATSUI, Shohei, Tokyo 103-8210 (JP); HIRANO, Takahiro, Tokyo 103-8210 (JP); HATSUTOMO, Taichi, Tokyo 103-8210 (JP)
(74) Representative: Jones, Nicholas Andrew
(86) International application number: PCT/JP2023/035314
(87) International publication number: WO 2024/090115

(57) **Abstract**

A solid personal care product includes a base part (10) and a liner portion (20) formed on the surface (10a) of the base part (10), in a plan view or an oblique downward view of the surface (10a), the change of the ratio (S1/S0) of the occupied area (S1) by the liner portion (20) per unit area (S0) is made along at least one direction (X) parallel with the surface (10a), and the change of the ratio of the occupied area (S1/S0) is configured to show a pattern using a subjective contour.

## Description

### Technical Field

The present invention relates to a solid personal care product and a method for producing a solid personal care product.

### Background Art

Patterns have been formed on solid personal care products such as soaps and solid cosmetics including eye shadows and foundations. For example, Patent Literature 1 discloses a cosmetic including an oily solid cosmetic packed and solidified in a container and including a pattern formed by discharging droplets of a decorative cosmetic onto the surface of the oily solid cosmetic. Patent Literature 2 discloses a solid cosmetic with a pattern. The solid cosmetic includes a three-dimensional pattern in a cosmetic dish, a transparent lower-layer cosmetic located below the pattern, and a transparent upper-layer cosmetic located over the pattern.

Patent Literature 3 discloses a cosmetic with a pattern. The cosmetic has a pattern on the surface of a cosmetic. In the cosmetic, the pattern includes first dots and second dots having a smaller area than that of the first dots, and combining a plurality of dots in the same color, including the first dots and the second dots, achieves color shades.

As for the technique for forming a pattern, Patent Literature 4 discloses a method of forming a relief pattern based on a line drawing. In the method, multiple reference lines are defined on a line drawing; the reference lines are bent near the intersections of a line constituting the line drawing and the reference lines; in a section where bending lines overlap, the lines are fused; the bending lines and some of the reference lines constitute multiparallel lines; and the multiparallel lines form a relief pattern.

### Citation List

### Patent Literatures

Patent Literature 1: JP 2019-172578 A
Patent Literature 2: JP 2019-108309 A
Patent Literature 3: JP 2017-86478 A
Patent Literature 4: JP H5-158208 A

### Summary of Invention

The present invention relates to a solid personal care product.

The solid personal care product preferably includes a base part and a liner portion formed on the surface of the base part.

In a plan view or an oblique downward view of the surface of the solid personal care product, a change of a ratio of an occupied area by the liner portion per unit area is preferably made along at least one direction parallel to the surface.

In the solid personal care product, the change of the ratio of the occupied area is preferably configured to show a pattern using subjective contours.

The present invention also relates to a method for producing a solid personal care product having a pattern based on an intended image.

The solid personal care product preferably includes a base part and a liner portion formed on the surface of the base part.

In a plan view or an oblique view of the solid personal care product, a change of a ratio of an occupied area by the liner portion per unit area is preferably made when the surface of the base part is viewed along a direction.

In the solid personal care product, the change of the ratio of the occupied area is preferably configured to show the pattern using subjective contours.

The production method preferably includes extracting, from an intended image, a measured values of pixels respective to points on a formation scheduled position of the liner portion, and forming the liner portion on the surface of the base part.

When forming the liner portion, referring to the measured values of the pixels extracted, the ratio of the occupied area is preferably changed by giving the occupied area respective to the measured values.

### Brief Description of Drawings

[Fig. 1] Fig. 1 is a perspective view schematically showing an embodiment of the solid personal care product of the present invention.
[Fig. 2] Fig. 2 is a plan view of the solid personal care product shown in Fig. 1.
[Fig. 3] Fig. 3 is an enlarged view of area A in Fig. 2.
[Fig. 4] Fig. 4 is a sectional view taken along line IV-IV in Fig. 3.
[Fig. 5] Fig. 5 is a view illustrating a method for determining the distance between the adjacent liner portions and is a schematic enlarged view of a part in Fig. 2.
[Fig. 6] Fig. 6(a) is a plan view showing a modified example of the solid personal care product shown in Fig. 1. Fig. 6(b) is a perspective view of the solid personal care product shown in Fig. 6(a) viewed from direction B. Fig. 6(c) is a perspective view of the solid personal care product shown in Fig. 6(a) viewed from direction C.
[Fig. 7] Figs. 7(a) to (d) are views showing modified examples of the arrangement pattern of the liner portion in the solid personal care product shown in Fig. 1.
[Fig. 8] Fig. 8 is a view showing another modified example of the arrangement pattern of the liner portion in the solid personal care product shown in Fig. 1.
[Fig. 9] Figs. 9(a) and (b) are views showing yet other modified examples of the arrangement pattern of the liner portion in the solid personal care product shown in Fig. 1.
[Fig. 10] Fig. 10 is a view showing a modified example of the solid personal care product shown in Fig. 1 and corresponding to Fig. 4.
[Fig. 11] Fig. 11 is a perspective view schematically showing an embodiment of a production apparatus used in the production method of the present invention.
[Fig. 12] Figs. 12 are views schematically illustrating the extraction step pertaining to the production method in the present embodiment. Fig. 12(a) is a view schematically showing a formation scheduled position of a liner portion. Fig. 12(b) is a view showing an example of an intended image used in the production method in the present embodiment. Fig. 12(c) is a view showing an intended image used in the production method in Example 2.
[Fig. 13] Figs. 13(a) to (c) are sectional views schematically showing a liner portion forming step in the production method in the present embodiment.
[Fig. 14] Fig. 14 is a sectional view schematically showing a liner portion forming unit pertaining to another embodiment of the production apparatus used in the production method of the present invention.
[Fig. 15] Figs. 15(a) and (b) are views showing yet other modified examples of the arrangement pattern of the liner portion in the solid personal care product shown in Fig. 1.

### Description of Embodiments

In the techniques disclosed in Patent Literatures 1 and 2, a cosmetic to constitute a pattern is ejected from a nozzle to form a pattern on the surface of a cosmetic (a lower-layer cosmetic in Patent Literature 2). In other words, in the techniques disclosed in Patent Literatures 1 and 2, the contour of a pattern is drawn to form the pattern itself. Accordingly, the techniques disclosed in Patent Literatures 1 and 2 are difficult to give shades to a pattern.

The technique in Patent Literature 3 enables the expression of color shades but is difficult to express a three-dimensional pattern.

The technique in Patent Literature 4 is used to form a relief pattern on stock certificates and other securities, and forming a pattern on a solid personal care product is not discussed in Patent Literature 4.

The present invention relates to a solid personal care product having a three-dimensional pattern capable of expressing shades.

The present invention will now be described with reference to preferred embodiments thereof.

A solid personal care product of the present invention preferably includes a base part 10 and a liner portion 20 formed on the surface 10a of the base part 10. Specifically, the solid personal care product preferably includes a main body 11 including the base part 10 and the liner portion 20.

The liner portion 20 preferably has a three-dimensional shape. Specifically, the liner portion 20 is preferably formed to protrude from the surface 10a of the base part 10.

The number of single liner portion 20 formed on the solid personal care product is preferably one. In other words, the liner portion 20 is formed as a single line. In this case, the liner portion 20 also preferably has a spiral shape which is being drawable with a single stroke.

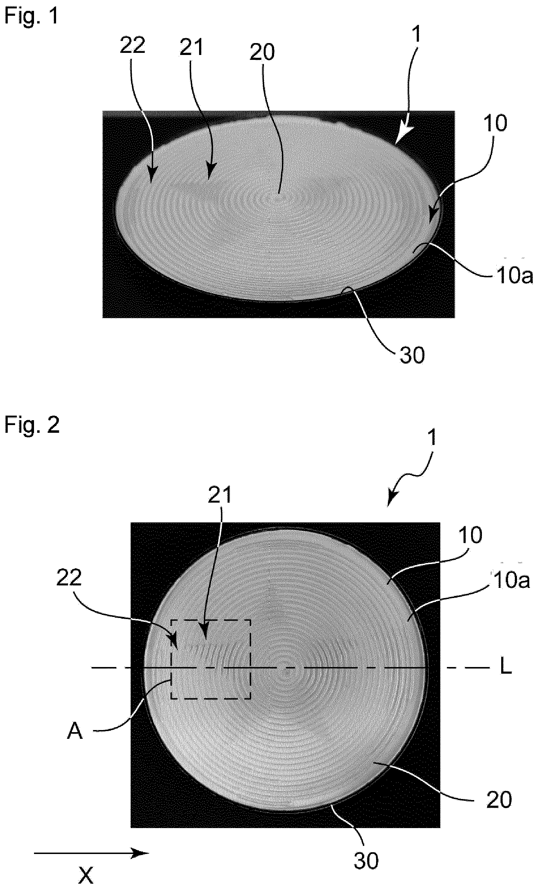

A typical example of the solid personal care product is a cosmetic. A cosmetic 1 preferably includes a main body 11 and a container 30 in which the main body 11 is stored therein. A specific example thereof is shown in Fig. 1.

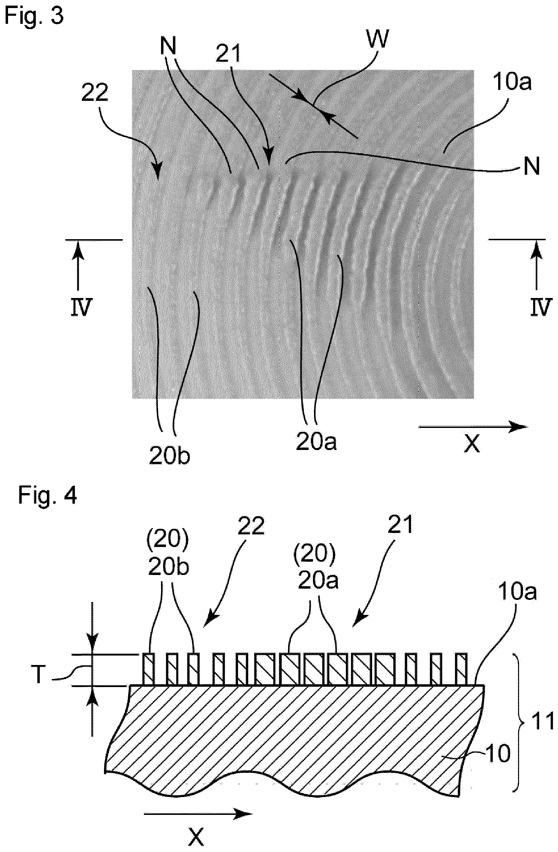

The three-dimensional liner portion 20 is a ridge formed on the surface 10a of the base part 10, and a specific example is shown in Fig. 4.

The solid personal care product may include two or more liner portions 20. In other words, the liner portion 20 may be partially broken.

In the solid personal care product of the present invention, the liner portion 20 preferably has a width W that changes along the longitudinal direction of the liner portion 20. The width W of the liner portion 20 may change continuously along the longitudinal direction of the liner portion 20 or may change in steps. The width W of the liner portion 20 preferably changes along at least one direction X parallel to the surface 10a of the base part 10 (see Fig. 2). The width W of the liner portion 20 may change continuously along one direction X or may change in steps. A specific example thereof is shown in Fig. 1 to Fig. 3.

In the example shown in the figures, the width W of the liner portion 20 continuously changes along the longitudinal direction of the liner portion 20.

In the example shown in the figures, the liner portion 20 has a portion having a width W that changes continuously along one direction X and has a portion having a width W that changes in steps along the one direction X.

In the solid personal care product of the present invention, the liner portion 20 preferably has a portion having a large width W (hereinafter also called a "wide portion") 20a and a portion having a small width W (hereinafter also called a "narrow portion") 20b. In the description, wide or narrow in width means a width relative to others. The liner portion 20 preferably has multiple wide portions 20a and multiple narrow portions 20b.

The solid personal care product typically has the wide portions 20a and the narrow portions 20b in a predetermined arrangement, and a sparse-dense structure including a dense portion 21 and a sparse portion 22 is formed in a plan view or an oblique downward view of the surface 10a of the base part 10. The dense portion 21 is an area in which the liner portion 20 is densely located on the surface 10a of the base part 10 or is an area in which the wide portions 20a are located. The sparse portion 22 is an area in which the liner portion 20 is sparsely located or is an area in which the narrow portions 20b are located.

Specifically, in a view of the surface 10a of the base part 10 viewed, for example, along one direction X, sparse portions 22 and dense portions 21 are alternately arranged. A sparse portion 22 is the portion in which the ratio of the area S1 occupied by a liner portion 20 per unit area S0 is relatively small in a plan view or an oblique downward view. A dense portion 21 is the portion in which the ratio of the area S1 occupied by a liner portion 20 per unit area S0 is relatively large in a plan view or an oblique downward view. The one direction X may be any direction parallel to the surface 10a of the base part 10.

Specific examples thereof are shown in Figs. 3 and 4.

As described above, in the solid personal care product of the present invention, the change of the ratio of the occupied area S1 occupied by the liner portion 20 per unit area S0, S1/S0, preferably is made along one direction X in a plan view or an oblique downward view of the surface 10a of the base part 10. The change of the occupied area ratio S1/S0 more preferably is made not only along one direction X but also along any direction parallel to the flat face of the base part 10. In other words, a change in the occupied area S1 is preferably observed in any view of the surface of the solid personal care product.

When a direction parallel with the flat face of the base part 10 is the longitudinal direction of the liner portion 20, the occupied area S1 changes as the width W changes. In this case, the exclusive area ratio changes at a changing point N at which the width W of the liner portion 20 changes. For example, a line connecting changing points N is the boundary between a dense portion and a sparse portion and is visually identified as a subjective contour.

Whether the change of the occupied area ratio S1/S0 is made along one direction parallel to the surface 10a of the base part 10 is determined by the following method.

### <Determination method>

First, an imaginary line is set along one direction parallel to the surface 10a of a base part 10. The one direction may be any direction parallel to the surface 10a. When the surface 10a of the base part 10 is viewed along the one direction, and a dense portion and a sparse portion of a liner portion exist on the surface 10a, or a sparse-dense structure is formed, the change of the occupied area ratio S1/S0 is determined to be made along the one direction.

At least in cases (1) to (4), a dense portion and a sparse portion of a liner portion are determined to exist.
(1) A portion in which parts of a liner portion are close to each other and the density is relatively high (dense portion) and a portion in which parts of the liner portion are separated from each other by a long distance and the density is relatively low (sparse portion) are included.

In this case, a liner portion preferably has a wide portion having a relatively large width and a narrow portion having a relatively small width, and accordingly the distance between parts of the liner portion changes. A liner portion having a wide portion and a narrow portion may be a single continuous liner portion (see Fig. 1) or multiple discontinuous liner portions. Multiple parallel liner portions, for example, two to twenty liner portions, also preferably have width-changing portions, such as width-increasing portions or width-decreasing portions, at the same position or near positions in the extending direction of the liner portions (see Fig. 1). In Fig. 3, N indicates a width-decreasing portion.

(2) A liner portion has a high portion with a relatively high height and a low portion with a relatively low height.

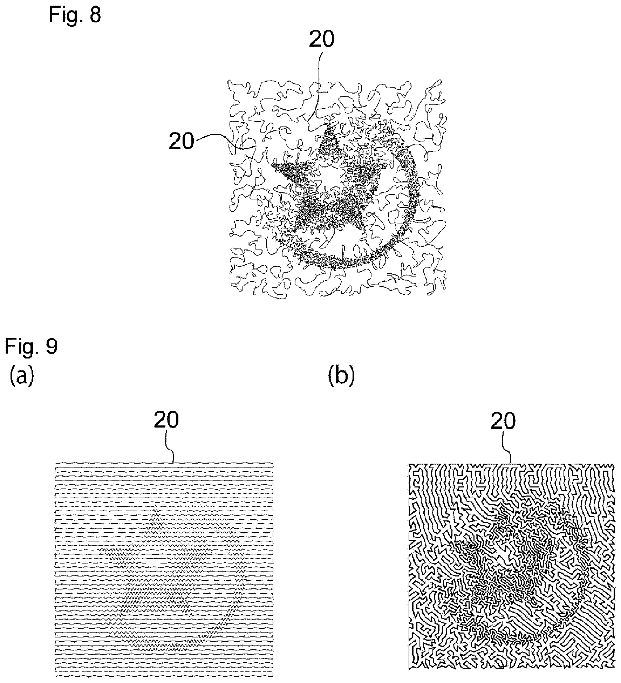

In this case, in an oblique downward view, the high portion forms a lot of shading and is visually identified as if the liner portion existed at a high density. Accordingly, the high portion becomes a dense portion. The low portion forms relatively less shading and is visually identified as if the liner portion existed at a low density. Accordingly, the low portion becomes a sparse portion. In the case of (2), a portion in which the high portion exists at a relatively high density (high-portion densely existing portion) and a portion in which the high portion exists at a relatively low density (high-portion sparsely existing portion) are preferably included.
(3) A portion in which a liner portion greatly bends and the total length of the liner portion in a certain area is large and a portion in which the liner portion less bends and the total length of the liner portion in a certain area is small are included (see Fig. 8 and Figs. 9(a) and (b)).
(4) Mixture of (1) to (3).

The occupied area ratio S1/S0 may be determined by the following method.

### <Determination method of occupied area ratio S1/S0>

The occupied area ratio S1/S0 in a plan view will be described.

In a plan view of the surface 10a of a base part 10, a region having a certain area is the region to be determined. The area of the region to be determined is the unit area S0. The plan view area of a liner portion in the region to be determined is determined. The plan view area of the liner portion determined as above is the occupied area S1 occupied by the liner portion. The occupied area S1 is divided by the unit area S0 to determine the ratio S1/S0.

Alternatively, the ratio S1/S0 may be determined by the following method. First, an image of a plan view of the surface 10a of a base part 10 is prepared, and, for example, a 10-mm square region is used as the region to be determined. The pixel number of the region to be determined is counted, and the counted pixel number is regarded as S0. The pixel number of the liner portion in the region to be determined is next counted, and the counted pixel number is regarded as S1. The pixel number S1 of the liner portion may be divided by the pixel number S0 of the region to be determined to determine the ratio S1/S0.

The region having a certain area to be determined may be appropriately set according to the fineness of a liner portion, the distribution, or the like, and may be, for example, a 10-mm square or 5-mm square region or a circular region having a diameter of 10 mm or a diameter of 5 mm. When a region to be determined has a higher definition, a 1-mm square or 0.5-mm square region or a circular region having a diameter of 1 mm or 0.5 mm may be used.

In the solid personal care product of the present invention, a change of the occupied area ratio S1/S0 is preferably configured to show a pattern using subjective contours. Specifically, the sparse-dense structure is preferably formed on the surface 10a of a base part 10 to form a pattern using subjective contours when the surface 10a is visually observed.

Examples shown in Figs. 1 and 2 are designed to form star and crescent patterns.

A subjective contour is the contour that is perceived by an optical illusion without any change in luminance or color along the contour line. In other words, a subjective contour is the physically absent contour that is perceived by a surrounding stimulus constellation and is also called an illusory contour, a cognitive contour, or a heterogeneous contour.

The subjective contour is widely known in various fields of images (JP 2010-4178 A, JP 2005-165060 A, and the like).

In the solid personal care product of the present invention, the change of the occupied area ratio S1/S0 or to be formed the sparse-dense structure on the surface 10a of the base part 10 is configured to show a pattern using subjective contours. Hence, the pattern of a solid personal care product can be made into a pattern with shades.

In the solid personal care product of the present invention, the liner portion 20 has a three-dimensional shape, and accordingly, the solid personal care product has a three-dimensional pattern. When the solid personal care product is visually observed, shading is formed.

The pattern of the solid personal care product of the present invention is formed by using subjective contours. In addition, shading is formed when the solid personal care product is visually observed. This can make the pattern more impressive or attractive.

As described above, the solid personal care product of the present invention can express a three-dimensional pattern which is capable to express shades.

In the solid personal care product of the present invention, instead of changing the width W of the liner portion 20 along one direction X or along the longitudinal direction of the liner portion 20, the height T of the liner portion 20 (see Fig. 4) may be changed along one direction X or along the longitudinal direction of the liner portion 20, and accordingly the change of the occupied area ratio S1/S0 may be made. Alternatively, both the width W and the height T of the liner portion 20 may be changed along one direction X or along the longitudinal direction of the liner portion 20, and accordingly the change of the occupied area ratio S1/S0 may be made.

Any one of or both of the width and the height of the liner portion 20 are changed along one direction X or along the longitudinal direction of the liner portion 20, and accordingly the change of the occupied area ratio S1/S0 is made. This can make visually observed shading clearer, and hence the pattern of the solid personal care product can be more impressive or attractive. In addition, the solid personal care product can be easily produced. A method for producing the solid personal care product will be described later.

The width W of the liner portion 20 in a plan view is not specifically limited, but is preferably 0.05 mm or more, more preferably 0.1 mm or more, and even more preferably 0.15 mm or more from the viewpoint of forming a three-dimensional pattern and of preventing a nozzle from clogging with a composition.

From the viewpoint of forming a high-definition pattern, the width W of the liner portion 20 is preferably 3 mm or less, more preferably 2 mm or less, and even more preferably 1.5 mm or less.

The width W of a liner portion 20 being within the above range means that both the width of the widest portion and the width of the narrowest portion of the liner portion 20 are within the above range.

In a plan view, the ratio of the width W2 of the narrowest portion of the liner portion 20 to the width W1 of the widest portion, W2/W1, is not specifically limited, but is preferably 1.6% or more, more preferably 2.5% or more, and even more preferably 3.3% or more from the viewpoint of making a pattern using subjective contours easily visible.

In a plan view, the ratio W2/W1 is preferably 90% or less, more preferably 80% or less, and even more preferably 70% or less from the viewpoint of forming a high-definition pattern. The ratio W2/W1 is expressed in percentage (%). The same is applied to the ratio T2/T1, the ratio D2/D1, the ratio D/W, the ratio R2/R1, and the ratio H2/H1 described later.

When a liner portion 20 is a ridge as in the embodiment, the height T of the liner portion 20 is not specifically limited, but is preferably 0.05 mm or more, more preferably 0.1 mm or more, and even more preferably 0.12 mm or more from the viewpoint of forming a three-dimensional pattern.

The height T of the liner portion 20 is preferably 2 mm or less, more preferably 1 mm or less, and even more preferably 0.6 mm or less from the viewpoint of preventing the liner portion from collapsing.

The height T of a liner portion 20 means the distance from the base to the tip of the liner portion 20 in the protruding direction of the liner portion 20 (see Fig. 4).

The height T of a liner portion 20 being within the above range means that both the height of the highest portion and the height of the lowest portion of the liner portion 20 are within the above range.

The ratio of the height T2 of the lowest portion of the liner portion 20 to the height T1 of the highest portion, T2/T1, is not specifically limited, but is preferably 2.5% or more, more preferably 5% or more, and even more preferably 8.3% or more from the viewpoint of making a pattern using subjective contours easily visible.

The ratio T2/T1 is preferably 90% or less, more preferably 80% or less, and even more preferably 70% or less from the viewpoint of forming a high-definition pattern.

The distance D between the adjacent liner portions 20 in a plan view is not specifically limited, but is preferably 0.15 mm or more, more preferably 0.5 mm or more, and even more preferably 1 mm or more from the viewpoint of preventing a previously formed liner portion 20 from coming into contact with a nozzle 61 in the liner portion forming step described later.

The distance D between the adjacent liner portions 20 in a plan view is preferably 15 mm or less, more preferably 10 mm or less, and even more preferably 5 mm or less from the viewpoint of making a pattern using subjective contours easily visible and of forming a high-definition pattern.

Of the distance D between the adjacent liner portions 20 in a plan view, the ratio of the shortest distance D2 to the longest distance D1, D2/D1, is preferably 5% or more, more preferably 10% or more, and even more preferably 20% or more from the viewpoint of easily forming a subjective contour due to the uniformity of the liner portion 20.

The ratio D2/D1 is preferably 100% or less, more preferably 90% or less, and even more preferably 80% or less from the viewpoint of easily preventing liner portions 20 from overlapping.

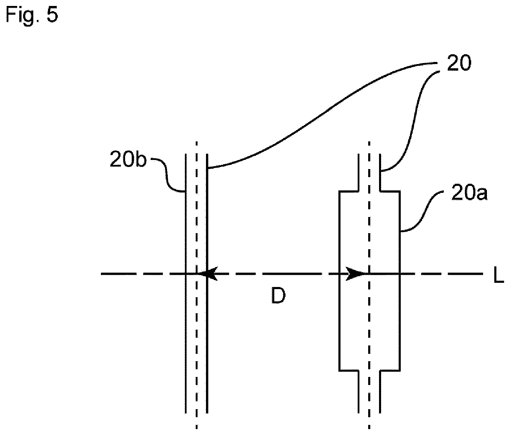

The distance D between the adjacent liner portions 20 is determined by the following method. First, an imaginary line L intersecting a liner portion 20 is set (see Fig. 2 and Fig. 5). For a cosmetic 1 having a single liner portion 20, the imaginary line L intersects the liner portion 20 at two or more places. For a cosmetic 1 having multiple liner portions 20, the imaginary line L may intersect one liner portion 20 of the multiple liner portions 20 at two or more places or may intersect two or more liner portions 20. Of the liner portion 20, the distance along the imaginary line L between the centers of the adjacent portions that intersect the imaginary line L is regarded as the distance D between the adjacent liner portions 20. Although the imaginary line L is preferably orthogonal to a liner portion 20, an imaginary line L orthogonal to a liner portion 20 cannot be set depending on the pattern, and the imaginary line L may be set at any position.

The distance D between the adjacent liner portions 20 being within the above range means that both the longest distance D and the shortest distance D between the liner portions 20 are within the above range.

In a plan view, the ratio of the distance D between the adjacent liner portions to the width W of the liner portion, D/W, is not specifically limited, but is preferably 5% or more, more preferably 7.5% or more, and even more preferably 10% or more from the viewpoint of preventing a previously formed liner portion 20 from coming into contact with a nozzle 61 in the liner portion forming step described later.

In a plan view, the ratio D/W is preferably 5 000% or less, more preferably 4 000% or less, and even more preferably 3 000% or less from the viewpoint of making a pattern using subjective contours easily visible and of forming a high-definition pattern.

To calculate the ratio D/W, the average width W of a liner portion is regarded as the width W of the liner portion, and the average distance D between the adjacent liner portions is regarded as the distance D between the adjacent liner portions.

In the solid personal care product of the present invention, a part including the base part 10 and the liner portion 20 preferably has a laminate structure in which two or more layers are laminated. A part having the laminate structure is preferably the part serving the product function when the solid personal care product is used. A part having such a laminate structure is called a functional part.

In the embodiment shown in Fig. 4, the base part 10 and the liner portion 20 are separate layers. In other words, in the embodiment, the main body 11 having a laminate structure in which the base part 10 and the liner portion 20 are laminated is the functional part.

When a part having a laminate structure has a laminate structure in which two or more layers are laminated, the respective layers can have separate functions, or a solid personal care product can have a more complicated pattern or have a more visible pattern. From the viewpoint of more markedly achieving the effects, the respective compositions constituting the layers in the laminate structure preferably differ from each other in any one or both of formulation and color.

When a part having a laminate structure has a laminate structure in which three or more layers are laminated, layers may have the same formulation or the same color. For example, in a functional part having a laminate structure in which three layers are laminated, two layers of the three layers may have the same formulation and the same color, and the two layers may differ from the remaining one layer in any one or both of formulation and/or color of the composition constituting each layer.

Between the liner portion 20 and the base part 10, another layer may be provided.

The liner portion 20 may have a laminate structure in which two or more layers are laminated, or the base part 10 may have a laminate structure in which two or more layers are laminated.

In the same layer, the composition may partially differ in any one or both of formulation and color. For example, when a liner portion 20 has partially different colors, the solid personal care product can have a pattern expressed in multiple colors, and this makes the pattern even more impressive or attractive.

In the solid personal care product of the present invention, the liner portion 20 is preferably formed of the layer most distant from the layer constituting the base part 10 in the part having a laminate structure. This structure makes the liner portion 20 easily visible, and a pattern using subjective contours can be easily identified. In the example shown in the figure, the layer most distant from the layer constituting the base part 10 is the layer constituting the surface that is to face a user using the solid personal care product.

The liner portion 20 may be formed of a layer other than the layer most distant from the layer constituting the base part 10 in the part having a laminate structure. In this case, a layer that is on the layer constituting the liner portion 20 and is opposite to the layer constituting the base part 10 is preferably transparent or translucent, and accordingly, the liner portion 20 is preferably visible when the solid personal care product is visually observed.

The solid personal care product of the present invention may be designed such that at least one of the hue contrast, the lightness contrast, and the saturation contrast of a pattern changes depending on the viewing direction. In the description, as for the "contrast" in hue contrast, lightness contrast, and saturation contrast, for example, the lightness will be described as an example: when the lightness of a base part 10 and the lightness of a liner portion 20 are simultaneously observed, the lightness of the liner portion 20 appears to be different due to the lightness effect of the base part 10. More specifically, when a base part 10 having a relatively high lightness and a liner portion 20 having a relatively low lightness are simultaneously observed, the lightness of the liner portion 20 appears lower than the true lightness of the liner portion 20. The same is applied to hue and saturation.

The mode in which a pattern has varying hue contrasts depending on the viewing direction includes a mode in which the hue of the pattern of a solid personal care product appears to be different when the pattern is viewed from a direction from when the pattern is viewed from another direction. In the description, varying hues means that hues are classified into different parts on a hue circle expressed in, for example, 40 equal parts. The mode in which a pattern has varying lightness contrasts depending on the viewing direction includes a mode in which the lightness of the pattern of a solid personal care product appears to be different when the pattern is viewed from a direction from when the pattern is viewed from another direction. The mode in which a pattern has varying saturation contrasts depending on the viewing direction includes a mode in which the saturation of the pattern of a solid personal care product appears to be different when the pattern is viewed from a direction from when the pattern is viewed from another direction.

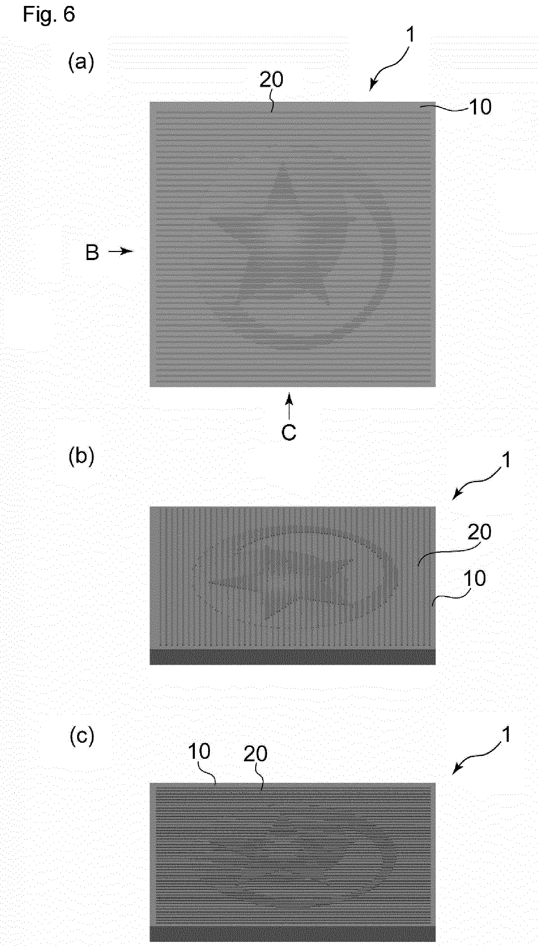

For example, a cosmetic 1 shown in Figs. 6(a) to (c) is designed such that the pattern has varying lightness contrasts depending on the viewing direction. Specifically, the cosmetic 1 shown in Fig. 6 is designed such that the lightness contrast of the pattern is relatively low when the cosmetic 1 is viewed from direction B (see Fig. 6(b)) and that the lightness contrast of the pattern is relatively high when the cosmetic 1 is viewed from direction C (see Fig. 6(c)). In other words, when the cosmetic 1 shown in Fig. 6 is viewed from direction B, the lightness of the pattern is perceived as relatively low, and the pattern shape is relatively less visible. When the cosmetic 1 is viewed from direction C, the lightness of the pattern is perceived as relatively high, and the pattern shape is relatively easily visible. Being viewed from direction B or direction C more specifically means that a face with the liner portion 20 of a solid personal care product is obliquely viewed from above.

A means for changing at least one of the hue contrast, the lightness contrast, and the saturation contrast of a pattern of the solid personal care product of the present invention depending on the viewing direction includes increasing the orientation of the liner portion 20 in a plan view of the solid personal care product.

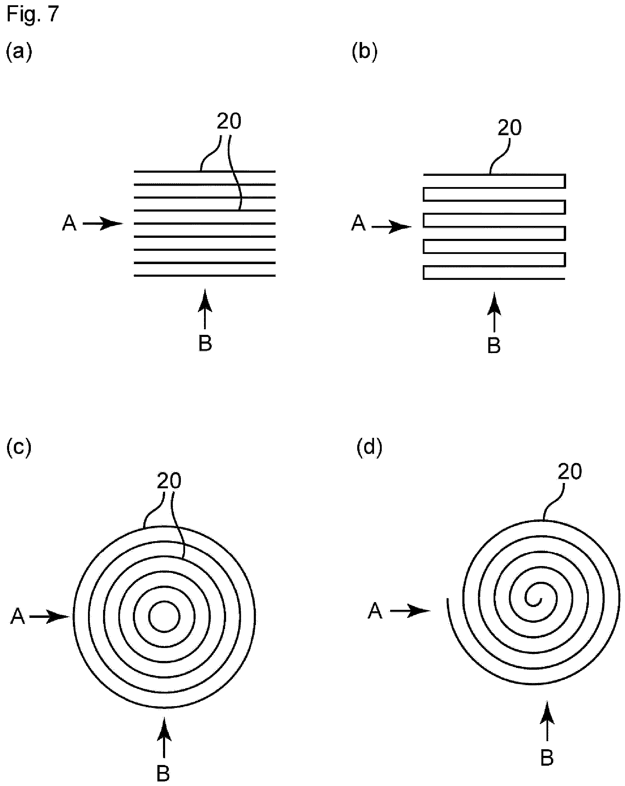

For example, when the arrangement pattern of a liner portion 20 is stripe (see Figs. 7(a) and (b)), the orientation of the liner portion 20 is high as compared with when the arrangement pattern of a liner portion 20 is concentric (see Fig. 7(c)) or is spiral (see Fig. 7(d)), and at least one of the hue contrast, the lightness contrast, and the saturation contrast of a pattern is likely to change depending on the viewing direction. In Figs. 7(a) to (c), the sparse-dense structure of the liner portion 20 is not shown for convenience of explanation.

In the examples shown in Figs. 7(a) and (b), the clearances between the liner portions 20 are easily visually identified when viewed from direction A as compared with when viewed from direction B. As described above in the examples shown in Figs. 7(a) and (b), the visibility of the clearances changes depending on the viewing direction. Hence, at least one of the hue contrast, the lightness contrast, and the saturation contrast of a pattern easily changes depending on the viewing direction as compared with the examples shown in Figs. 7(c) and (d).

The orientation of a liner portion 20 can be quantitatively evaluated as follows: an image of a plan view of a solid personal care product is prepared; and the image is subjected to Fourier transformation image processing. Specifically, by Fourier transformation, a power spectrum is prepared, and the angular distribution of the amplitude is calculated. From the angular distribution of the amplitude, the orientation intensity is calculated, and the orientation intensity can be regarded as an index of orientation. The orientation intensity is calculated as the major axis/minor axis ratio of an approximate ellipse in an angular distribution diagram of amplitude. A liner portion having an orientation intensity of not more than 1.1 is determined to have no orientation, a liner portion having an orientation intensity of more than 1.1 and less than 1.2 is determined to have weak orientation, and a liner portion having an orientation intensity of not less than 1.2 is determined to have strong orientation.

The solid personal care product of the present invention may have a single liner portion 20 (see Fig. 1, Fig. 7(b), and Fig. 7(d) or may have two or more liner portions 20 (see Fig. 7(a) and Fig. 7(c)).

In a solid personal care product having a single liner portion 20, the terminal points of the liner portion 20 are only the start point and the end point. When a composition M is discharged from a nozzle 61 to form a liner portion 20 in the liner portion forming step described later, the width W of the liner portion 20 is likely to vary involuntarily at the terminal point such as the start point and the end point. To produce a cosmetic having a single liner portion 20, the liner portion 20 is easily formed as intended on the surface 10a of a base part 10, and the cosmetic 1 can be easily produced.

When having multiple liner portions 20, the cosmetic 1 can have more various patterns. Each liner portion 20 may be a single line having a start point and an end point or may be a circle having no start point or no end point. In the description, a liner portion 20 being a circle having no start point or no end point means that the liner portion 20 is a circle having no start point or no end point in a plan view or an oblique downward view of the liner portion 20 formed in a solid personal care product. The liner portion 20 as a circle having no start point or no end point may have, for example, a formation start point that is initially formed and a formation end point that is finally formed when the liner portion 20 is formed. For a solid personal care product having multiple liner portions 20, the number of liner portions 20 is not limited.

The liner portion 20 may be arranged so as not to intersect a part of the liner portion 20 or another liner portion 20 different from the liner portion 20 (see Fig. 1 and Figs. 7(a) to (d)) or may be arranged so as to intersect a part of the liner portion 20 or another liner portion 20 different from the liner portion 20 (see Fig. 8).

When a composition M is discharged from a nozzle 61 to form a liner portion 20 in the liner portion forming step described later, the width W of the liner portion 20 is likely to vary involuntarily at intersections between parts of the liner portion 20 or at intersections between the liner portion 20 and another liner portion 20 different from the liner portion. From the viewpoint of suppressing such an involuntary variation of the width W of the liner portion 20, the liner portion 20 is preferably arranged so as not to intersect a part of the liner portion 20 or another liner portion 20 different from the liner portion 20.

From the viewpoint of diversifying the expression manner of a pattern of the solid personal care product, the liner portion 20 is preferably arranged so as to intersect a part of the liner portion 20 or another liner portion 20 different from the liner portion 20. When the liner portion 20 is arranged so as to intersect a part of the liner portion 20 or another liner portion 20 different from the liner portion 20, the number of intersections is not specifically limited.

In each example shown in Fig. 1, Fig. 7(b), and Fig. 7(d), the liner portion 20 is arranged so as not to intersect any part of the liner portion 20.

In the examples shown in Fig. 7(a) and Fig. 7(c), each liner portion 20 is arranged so as not to intersect any part of the liner portion 20, and the respective liner portions 20 are arranged so as not to intersect each other.

In the solid personal care product of the present invention, instead of changing the width W of the liner portion 20, the liner portion 20 may curve, and accordingly the change of the occupied area ratio S1/S0 may be made (see Fig. 9(a)). The liner portion 20 may have a single curving portion or have a plurality of curving portions.

The liner portion 20 may bend, and accordingly the change of the occupied area ratio S1/S0 may change (see Fig. 9(b)). The liner portion 20 may have a single bending portion or have multiple bending portions.

The liner portion 20 may curve and bend, and accordingly the change of the occupied area ratio S1/S0 may be made. The liner portion 20 may have a single curving portion and a single bending portion, have a single curving portion and multiple bending portions, have multiple curving portions and a single bending portion, or have multiple curving portions and multiple bending portions.

From the viewpoint of simplifying the control of the width W of the liner portion 20, of gradually moving the relative position of an object 70 to a nozzle 61 in the liner portion forming step described later, and of suppressing vibration when the liner portion is drawn, the liner portion 20 preferably curves, and accordingly the change of the occupied area ratio S1/S0 is preferably made.

From the viewpoint of simplifying the control of the width W of the liner portion 20 and of forming a high-definition pattern, the liner portion 20 preferably bends, and accordingly the change of the occupied area ratio S1/S0 is preferably made.

From the viewpoint of simplifying the control of the width W of the liner portion 20 and of diversifying the expression manner of a pattern, the liner portion 20 preferably curves and bends, and accordingly the change of the occupied area ratio S1/S0 is preferably made.

For a curving liner portion 20, the curvature radius R of the liner portion 20 in a plan view is not specifically limited, but is preferably 0.1 mm or more, more preferably 0.2 mm or more, and even more preferably 0.3 mm or more from the viewpoint of preventing a previously discharged liner portion from coming into contact with a nozzle.

The curvature radius R of the liner portion 20 in a plan view is preferably 1 000 mm or less, more preferably 800 mm or less, and even more preferably 600 mm or less from the viewpoint of making a pattern using subjective contours easily visible and of forming a high-definition pattern.

The curvature radius R of a liner portion 20 being within the above range means that both the curvature radius at a position having the largest curvature radius of the liner portion 20 and the curvature radius at a position having the smallest curvature radius are within the above range.

In a plan view, the ratio of the curvature radius R2 at a position having the smallest curvature radius of the liner portion 20 to the curvature radius R1 at a position having the largest curvature radius, R2/R1, is not specifically limited, but is preferably 0.01% or more, more preferably 0.02% or more, and even more preferably 0.03% or more from the viewpoint of preventing a previously discharged liner portion from coming into contact with a nozzle.

In a plan view, the ratio R2/R1 is preferably 90% or less, more preferably 80% or less, and even more preferably 70% or less from the viewpoint of making a pattern using subjective contours easily visible and of forming a high-definition pattern.

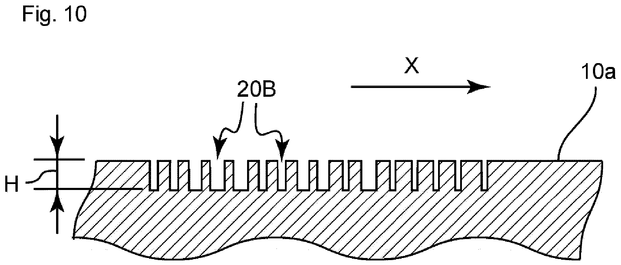

In the solid personal care product of the present invention, the liner portion 20 may be a groove 20B formed on the surface 10a of the base part 10 in place of a ridge formed on the surface 10a of the base part 10. In other words, the liner portion 20B may be formed by grooving the surface 10a of the base part 10. A specific example thereof is shown in Fig. 10.

When a liner portion is a groove 20B, the depth H of the liner portion 20B (see Fig. 10) is not specifically limited, but is preferably 0.05 mm or more, more preferably 0.1 mm or more, and even more preferably 0.12 mm or more from the viewpoint of forming a three-dimensional pattern.

The depth H of the liner portion 20B is preferably 2 mm or less, more preferably 1 mm or less, and even more preferably 0.6 mm or less from the viewpoint of preventing an object 70 between the adjacent liner portions 20B from collapsing.

The depth H of a liner portion 20B means the distance in the depth direction of the liner portion 20 from the surface 10a of the base part 10 to the bottom face of the liner portion 20B (see Fig. 10).

The depth H of a liner portion 20B being within the above range means that both the depth of the deepest portion of the liner portion 20B and the depth of the shallowest portion are within the above range.

The ratio of the depth H2 of the shallowest portion of the liner portion 20B to the depth H1 of the deepest portion, H2/H1, is not specifically limited, but is preferably 2.5% or more, more preferably 5% or more, and even more preferably 8.3% or more from the viewpoint of making a pattern using subjective contours easily visible.

The ratio H2/H1 is preferably 90% or less, more preferably 80% or less, and even more preferably 70% or less from the viewpoint of forming a high-definition pattern.

A preferred first embodiment of a method for producing the solid personal care product of the present invention will next be described with reference to a method for producing the cosmetic 1 shown in Figs. 1 to 5.

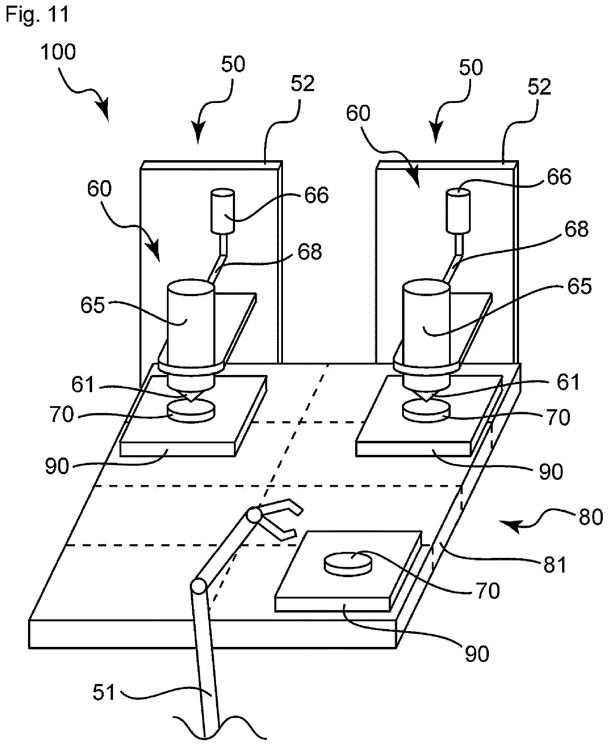

A production apparatus 100 suitably used for the production method in the first embodiment typically includes an extraction unit (not shown) and a liner portion forming unit 50 configured to form a liner portion 20 on the surface 10a of a base part 10 of a solid personal care product. The production apparatus 100 may further include a conveyor unit 80. A specific example thereof is shown in Fig. 11.

The extraction unit is typically configured to extract measured values of pixels 45, 46, 47 respective to points 25, 26, 27 on the formation scheduled position (hereinafter also called "path") 24 of a liner portion 20 from an intended image. Specifically, the extraction unit can use image processing software, matrix calculation software, spreadsheet software, or image processing programs installed in a computer to extract measured values of pixels 45, 46, 47 respective to points 25, 26, 27 on the path 22 of a liner portion 20 from an intended image.

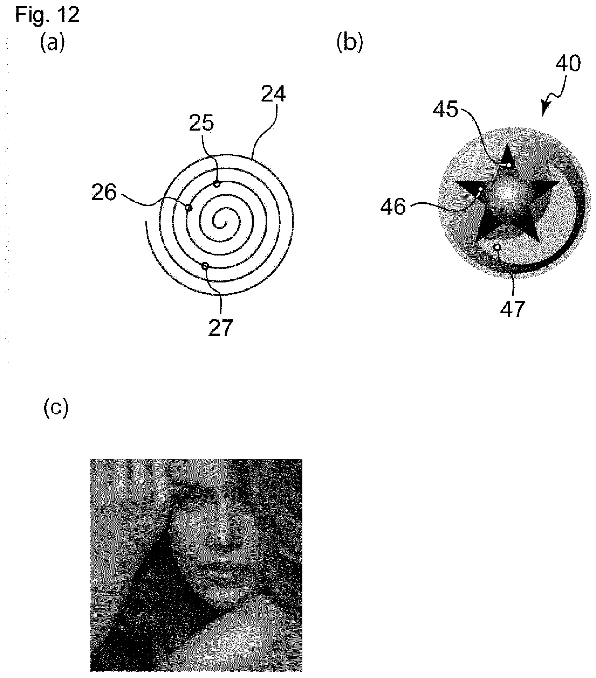

The planar shape of a path 24 respective to the planar shape of the liner portion 20 of a solid personal care product to be produced. In the specific example shown in Fig. 12, the planar shape of the path 24 is spiral.

The liner portion forming unit 50 typically includes a supply unit 60 configured to supply a flowable composition M to an object to which the composition is to be supplied (hereinafter also simply called an object) 70 and a nozzle 61 integrally provided so as to communicate with the supply unit 60. In the liner portion forming unit 50, typically, a plate-shaped object placement unit 90 is provided below the nozzle 61 and to face the nozzle 61, and an object 70 can be loaded or fixed onto the top face of the object placement unit 90.

In the first embodiment, the object 70 is a cosmetic 1 before formation of a liner portion 20 on the surface 10a of the base part 10. In the present embodiment, the composition M discharged from the nozzle 61 is deposited on the surface 10a of the base part 10 as the object 70 to form a liner portion 20.

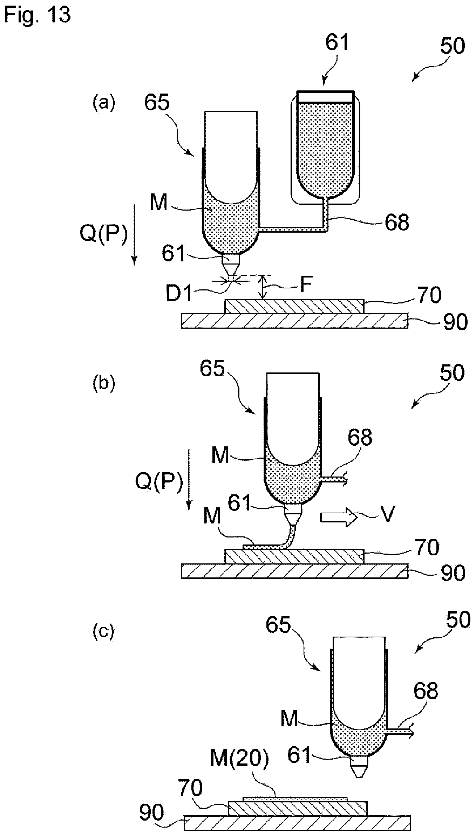

The liner portion forming unit 50 preferably includes a position adjustment unit 52 configured to support or hold the supply unit 60 and the object placement unit 90 at envisaged positions. A specific example thereof is shown in Fig. 11 and Fig. 13.

The position adjustment unit 52 preferably has a position adjustment mechanism that relatively transfers the position of the nozzle 61 and the position of the object placement unit 90 in any direction. Accordingly, at least one of the nozzle 61 and the object placement unit 90 can be transferred in a planar direction, a vertical direction, or a combination direction thereof, and at least one of the nozzle 61 and an object 70 on the object placement unit 90 can be transferred relative to the other. The position adjustment mechanism may be manually operated or be automatically controlled in response to electric signals from a controller or the like.

The supply unit 60 is a member configured to send a flowable composition M to an object 70. The supply unit 60 preferably includes a liquid sending unit 65 and a composition storing unit 66.

The liquid sending unit 65 is preferably connected to communicate with the composition storing unit 66 through a flow passage 68. Accordingly, a composition M supplied from the composition storing unit 66 into the liquid sending unit 65 can be supplied to the nozzle 61 continuously or discontinuously, or the supply can be stopped.

As such a liquid sending unit 65, a jet dispenser capable of discharging droplets of a composition M, or a mohno dispenser or a screw dispenser capable of continuously discharging a composition M can be used.

One end of the composition storing unit 66 is preferably connected to a pump or a pressurizing means such as air such that the composition M stored in the composition storing unit 66 can be forced to send through the flow passage 68 to the liquid sending unit 65.

The nozzle 61 is typically a tubular member for supplying a composition M from the supply unit 60 toward an object 70. In the nozzle 61, a flow passage of a composition M is formed in the space thereof along the flow direction P of the composition M. A nozzle tip as one end of the nozzle 61 constitutes a supply port for a composition M, and the other end is connected to communicate with the above supply unit 60.

The constituent material of the nozzle is not specifically limited, and, for example, a metal or a plastic may be used.

The conveyor unit 80 is typically configured to convey the object placement unit 90 to a position below the nozzle 61 and facing the nozzle 61. Specifically, the conveyor unit 80 includes the object placement unit 90, a stage 81, and a controller (not shown).

In the production apparatus 100, an object supplying and collecting unit 50 preferably has a manipulator robot 51. The manipulator robot 51 is, for example, a robot arm and is configured to grip an object 70. The manipulator robot 51 is also configured to release the gripping state of an object 70 above the object placement unit 90 to place the object 70 on the object placement unit 90. The manipulator robot 51 in a non-gripping state is configured to grip an object 70 on the object placement unit 90 to collect the object. A specific example thereof is shown in Fig. 11.

In the production apparatus 100, the conveyor unit 80 preferably constitutes what is called a "linear conveyor system". Specifically, the conveyor unit 80 preferably includes a stage 81, object placement units 90, and a controller (not shown). Each object placement unit 90 typically includes a permanent magnet. The stage 81 typically includes a segment containing a magnet coil therein. The magnet coil in the stage 81 is energized to generate a magnetic field, and accordingly the object placement unit 90 floats on the stage 81 and can move freely. A specific example thereof is shown in Fig. 11.

The stage 81 may include a single segment or may include multiple segments. For a stage 81 including multiple segments, each segment is typically a physically independent unit, and each unit contains a magnet coil therein. Segments may be arranged continuously in one direction, but are preferably arranged two-dimensionally to be connected to each other and to constitute the stage 81. In the production apparatus 100 shown in Fig. 11, the stage 81 includes eight segments.

The respective stages 81 of the conveyor unit 80 can independently, freely convey object placement units 90. Hence, the object placement units 90 conveyed by the respective stages 81 can be conveyed on different conveying pathways and can be conveyed to positions below different nozzles 61 and facing the corresponding nozzles 61. In other words, solid personal care products as objects 70 can be subjected to different liner portion forming steps. Liner portions 20 formed in different liner portion forming steps may form different patterns or may form the same pattern.

The production method of the present invention is a method for producing a solid personal care product having a pattern based on an intended image.

The production method of the present invention preferably includes an extraction step of extracting, from an intended image, predetermined values of pixels 45, 46, 47 corresponding to a plurality of points 25, 26, 27 on a path 24 and includes a liner portion forming step of forming a liner portion 20 on the surface 10a of a base part 10.

The production method of the present invention preferably includes, before the extraction step, a converting of a color tone (a color tone conversion step) and a setting of interest region (interest region setting step). The color tone conversion step may be included after the extraction step.

The production method of the present invention may include, before the liner portion forming step, a conveyance step of conveying an object placement unit 90 with an object 70 to a position below a nozzle 61 and facing the nozzle 61.

In the color tone conversion step, an intended image is converted into a envisaged color tone. Examples of the color tone conversion method include binarizing, gray scaling, and monochromatizing. Before the color tone conversion step, an intended image may be subjected to processing such as gamma correction and contrast control. After the color tone conversion step, processing such as denoising may be performed.

The interest region setting step may be performed before the color tone conversion step or after the color tone conversion step. In the interest region setting step, a region to be formed into a pattern is set from an intended image. For example, unnecessary regions may be removed from an intended image to leave only the region to be formed into a pattern in the image. Regions to be formed into a pattern may be selected from an intended image, and the selected regions may be rearranged to prepare a region to be formed into a pattern. An intended image may be subjected to enlargement processing or reduction processing for changing the image size. The enlargement processing or reduction processing may be performed while the aspect ratio of an image is fixed or while the aspect ratio is changed.

In the extraction step, the extraction unit is used to extract, from an intended image 40, predetermined values of pixels 45, 46, 47 corresponding to a plurality of points 25, 26, 27 on a path 24 (see Fig. 12). The pixels indicated by signs 45, 46, 47 in Fig. 12(b) are pixels corresponding to the points indicated by signs 25, 26, 27, respectively, in Fig. 12(a). A pixel predetermined value may be, for example, luminance, hue, lightness, or saturation.

For example, when an intended image is binarized in the color tone conversion step, a measured value of a pixel extracted in the extraction step is luminance expressed in two gradations of 0 and 255. When an intended image is gray-scaled in the color tone conversion step, a measured value of a pixel extracted in the extraction step is luminance expressed in 256 gradations from 0 to 255. To obtain hue, an image is converted to grayscale or two gradations according to the color code in the color tone conversion step, and then the extraction step is performed.

The extraction step will be described in detail. In the extraction step, a path image having a path according to the pixel numbers of an intended image is created. In the step, the path image is, for example, a binary image. A coordinate corresponding to the path is set at a luminance of 0, and a coordinate not corresponding to the path is set at a luminance of 1. At a coordinate (i,j) where a pixel P (i,j) is a luminance of 0 in a path image, a measured value of pixel I (i,j), such as luminance, hue, lightness, or saturation, is obtained in the corresponding intended image. The pixel P is any pixel selected from a path image. The pixel I is pixel in an intended image and is the pixel corresponding to the pixel P in a path image in the intended image. When the size of a path image differs from the size of an intended image, an image size adjustment step of adjusting the size of a path image or the size of an intended image may be performed. The image size adjustment step may be performed before the extraction step or after the extraction step. For example, when a path image is smaller than an intended image, the image size adjustment step may be performed to reduce the intended image to the size of the path image for size adjustment. When a path image is larger than an intended image, the image size adjustment step may be performed to enlarge the intended image to the size of the path image for size adjustment. In the image size adjustment step, typically, the size of an intended image is adjusted to fit the size of a path image, but a path image may be enlarged or reduced to fit the size of an intended image.

In Fig. 12, for convenience of explanation, points 25, 26, 27 on the path 24 and three pixels 45, 46, 47 corresponding to the three points 25, 26, 27 are shown, but the numbers of points and pixels are not specifically limited.

To one point on a path 24, only one pixel may correspond, or multiple pixels may correspond. For example, one point on a formation scheduled position 22 of a liner portion 20 may be a region including multiple pixels in an intended image. In this case, the average of the measured values of the pixel may be a measured value at one point on the formation scheduled position 22 of a liner portion 20. The average may be a value rounded to a whole number.

In the extraction step, a path image having a path according to the pixel number of an intended image may be created; and instead of obtaining a measured value of pixel I (i,j) in the intended image, a drawing having a path may be programmed (for example, G-coded) to determine a measured value of pixel I (i,j) in the intended image corresponding to the coordinate (i,j) in the program.

In the conveyance step, the object placement unit 90 with an object 70 is conveyed to a position below the nozzle 61 and facing the nozzle 61. Specifically, first, the magnet coil in a stage 81 is preferably energized to generate a magnetic field, and an object placement unit 90 is preferably allowed to float. A manipulator robot 51 is then preferably used to place an object 70 on the object placement unit 90. The controller (not shown) of a conveyor unit 80 is then preferably used to control the position of the object placement unit 90. Specifically, the object placement unit 90 is preferably conveyed to a position below the nozzle 61 and facing the nozzle 61.

In the liner portion forming step, a liner portion 20 is formed on the surface 10a of a base part 10. Specifically, a composition M to constitute a liner portion 20 is preferably discharged toward an object 70 on the object placement unit 90 conveyed in the conveyance step and is preferably deposited on the object to form a liner portion 20. In the step, while the composition M is supplied from the nozzle 61 toward the object 70 (see Fig. 13(a)), at least one of the nozzle 61 and the object 70 is preferably transferred relative to the other (see Fig. 13(b)) to create a deposit derived from the composition or a liner portion 20 on the object 70. In the liner portion forming step, a flowable composition M is preferably discharged from the nozzle 61.

When at least one of the nozzle 61 and the object 70 is transferred relative to the other, the relative position of the nozzle 61 to the object 70 preferably moves along a path 24. In the first embodiment, at least one of the nozzle 61 and the object 70 is preferably transferred relative to the other such that a liner portion 20 is formed spirally in a plan view.

The relative transfer direction of the nozzle 61 may be transferred in a planar direction, a vertical direction, or a combination direction thereof according to the planar shape or three-dimensional shape of an intended liner portion 20 or to a drawn pattern.

A pattern in the specification of the present application means, for example, various characters such as Japanese characters including hiragana and katakana, alphabets, Arabic numerals, Roman numerals, and any foreign characters, graphic patterns and geometric shapes formed from straight lines, curved lines, or a combination thereof, and shapes of symbols, colors, designs, or a combination thereof.

Typically, a pattern is formed from a liner portion 20. A narrow portion 20b forms a graphic pattern such as a spiral pattern or a geometric shape pattern; and a pattern from a wide portion 20a or a pattern using subjective contours forms various characters, a graphic pattern or a geometric shape from straight lines, curved lines, and a combination thereof, and shapes of symbols, colors, designs, or a combination thereof.

Such a pattern is preferably colored in a color identical or different from the original ground color of an object 70 to which the pattern is to be provided and thus is preferably made visible.

In the liner portion forming step, according to a measured value of a pixel extracted in the extraction step, the occupied area ratio S1/S0 is preferably changed by giving an occupied area S1 respective to the measured value of the pixel. For example, when a composition M is supplied from the nozzle 61 toward an object 70, at least one of the relative transfer speed V of the nozzle 61 to the base part 10, the distance F between the base part 10 and the nozzle 61, and the supply speed Q of the composition M supplied from the nozzle 61 toward the surface 10a of the base part 10 is changed according to a measured value of a pixel extracted in the extraction step, and thus the occupied area S1 respective to the measured value can be given. By changing at least one of the relative transfer speed V, the distance F, and the supply speed Q to change the width W of a liner portion 20 formed on an object 70, the occupied area S1 can be made respective to the measured value. Instead of changing the width W of a liner portion 20 formed on an object 70, the height T of the liner portion 20 may be changed, or both the width W and the height T of the liner portion 20 may be changed.

An example in which the relative transfer speed V is changed will next be described. For example, when an intended image is binarized in the color tone conversion step, the measured value is expressed in two gradations of 0 and 255. Referring to the measured value, the relative transfer speed V may be controlled in two levels of low speed and high speed. The relative transfer speed V may be set to low speed when the measured value is 0, and the relative transfer speed V may be set to high speed when the measured value is 1, or vice versa. When the relative transfer speed V is low speed, a portion respective to the wide portion 20a in a liner portion 20 is formed, and when the relative transfer speed V is high speed, a portion respective to the narrow portion 20b in the liner portion 20 is formed.

When an intended image is gray-scaled in the color tone conversion step, the measured value is expressed in 256 gradations from 0 to 255. For example, the measured values may be divided into three levels of 0 to 100, 101 to 200, and 201 to 255. When the measured value is 0 to 100, the relative transfer speed V may be set to low speed; when the measured value is 101 to 200, the relative transfer speed V may be set to medium speed; and when the measured value is 201 to 255, the relative transfer speed V may be set to high speed. The measured values may be divided into any levels. The measured values may be divided into two levels or into 256 levels. The threshold levels for dividing the measured values into multiple levels are not also limited.

In accordance with the image tone conversion system, the gradations of a 256-gradation image may be limited to two gradations, four gradations, eight gradations, 16 gradations, or the like to set the speed. For example. when an image is converted into two gradations, the relative transfer speed V may be set to low speed for a portion having a measured value of 0 to 127, and the relative transfer speed V may be set to high speed for a portion having a measured value of 128 to 255. In a similar manner, when an image is converted into four gradations, measured values may be divided into, for example, 0 to 63, 64 to 127, 128 to 191, and 192 to 255, and the speed may be changed in four levels from low to slow. The same is applied to cases of eight gradations, 16 gradations, and the like.

When the distance F or the supply speed Q is changed, a similar procedure to that for the relative transfer speed V may be performed.

The relative transfer speed V, the distance F, or the supply speed Q may be changed continuously or be changed in steps.

Finally, supplying the composition M from the nozzle 61 is stopped (see Fig. 13(c)). Through the steps, a liner portion 20 having a envisaged shape is formed on the object 70.

Accordingly, for example, a liner portion 20 formed by using a cosmetic slurry can be a three-dimensional object including the liner portion containing a cosmetic derived from the composition.

As needed, the nozzle 61 may be relatively transferred on the same plane to the object 70 or may be relatively separated.

By forming a liner portion 20 on the surface 10a of a base part 10 as described above, the solid personal care product of the present invention is produced. The solid personal care product produced as above may be directly used as an intended personal care product or may be subjected to another step to yield an intended personal care product. In any case, by the production method of the present invention, an intended high-definition design can be drawn with high accuracy from the original formulation of a composition, without any mold or the like. This enables simple and efficient shaping and decoration with good shape or dimensional accuracy.

In the liner portion forming step, according to a pixel predetermined value extracted in the extraction step, an occupied area S1 corresponding to the predetermined value is given to change the occupied area ratio S1/S0. Hence, a liner portion 20 creating a pattern using subjective contours can be efficiently produced, and this enables efficient production of a cosmetic 1 having a three-dimensional pattern which is capable to express shades.

The relative transfer speed V is not specifically limited, but is preferably 1 mm/min or more, more preferably 2 mm/min or more, and even more preferably 3 mm/min or more from the viewpoint of improving the production capacity.

The relative transfer speed V is not specifically limited as long as the supply can be performed, but is preferably 5 000 mm/min or less, more preferably 500 mm/min or less, and even more preferably 200 mm/min or less from a practical viewpoint.

When the line width is changed, the relative transfer speed V is preferably 10 mm/min or more, more preferably 50 mm/min or more, and even more preferably 300 mm/min or more and is preferably 60 000 mm/min or less, more preferably 10 000 mm/min or less, and even more preferably 3 000 mm/min or less.

The relative transfer speed V may be appropriately changed, for example, by changing the driving speed of the above position adjustment unit 52. The relative transfer speed may also be appropriately changed by changing the conveyance speed of the object placement unit 90 with an object 70.

In the specification of the present application, the relative transfer speed V is expressed as an absolute value.

The distance F is not specifically limited, but is preferably 0.05 mm or more, more preferably 0.1 mm or more, and even more preferably 0.15 mm or more from the viewpoint of preventing the nozzle 61 from coming into contact with an object 70.

The distance F is preferably 5 mm or less, more preferably 2.5 mm or less, and even more preferably 2 mm or less from the viewpoint of improving the following performance of a discharged composition M to the relative movement of the nozzle 61 and an object 70.

The supply speed Q is not specifically limited, but is preferably 0.05 mm³/s or more, more preferably 0.1 mm³/s or more, and even more preferably 0.4 mm³/s or more from the viewpoint of improving the production capacity.

The supply speed Q is not specifically limited as long as the operation can be performed, but is preferably 150 mm³/s or less, more preferably 50 mm³/s or less, and even more preferably 15 mm³/s or less from the viewpoint of stably forming a high-definition liner portion due to the balance with the relative transfer speed of the nozzle 61.

The supply speed Q can be appropriately changed by appropriately adjusting the rotation rate of a rotor when, for example, the above dispenser is used as the liquid sending unit 65 in the supply unit 60, or by adjusting the shape or inner diameter of the nozzle 61 or the viscosity or the like of a composition M.

In the liner portion forming step, a composition M to constitute a liner portion 20 is preferably discharged from the nozzle 61 toward the surface 10a of a base part 10 and is preferably deposited on the surface to form a liner portion 20. This enables easy formation of a liner portion 20.

A second embodiment of the production method of the present invention will next be described.

The production method in the second embodiment will be described mainly on different points from the production method in the first embodiment. To the points not specifically described, the above description for the first embodiment will be appropriately applied.

In the production method of the present invention, in the liner portion forming step, instead of discharging a composition M from the nozzle 61 toward the surface 10a of a base part 10 and depositing the composition on the surface, a part of the surface 10a of a base part 10 may be engraved to form a liner portion 20B. In the production method, the formed liner portion 20B is a groove.

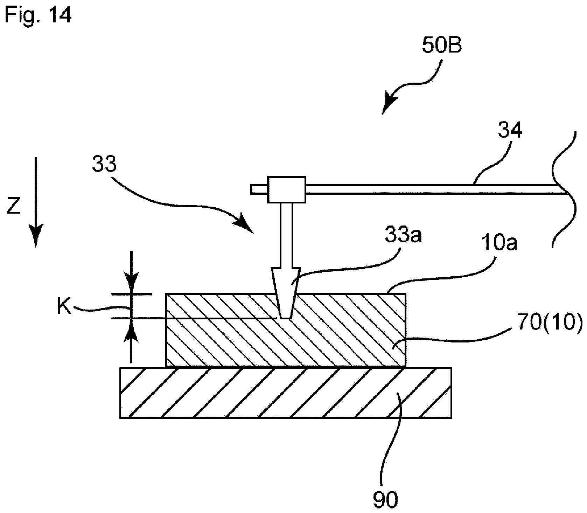

A liner portion forming unit 50B of a production apparatus 100 preferably used in the production method in the second embodiment preferably includes an engraving tool 33 and a position adjustment unit 34 for supporting or holding the engraving tool 33 at a predetermined position. An example of the liner portion forming unit 50B is shown in Fig. 14

The position adjustment unit 34 preferably includes a position adjustment mechanism that relatively transfers the position of the engraving tool 33 in any direction. Accordingly, at least one of the engraving tool 33 and the object placement unit 90 can be transferred in a planar direction, a vertical direction, or a combination direction thereof, and at least one of the engraving tool 33 and an object 70 on the object placement unit 90 can be transferred relative to the other. The position adjustment mechanism may be manually operated or be automatically controlled in response to electric signals from a controller or the like.

The engraving tool 33 typically has an enlarged diameter part 33a that is at least a part of the tip of the engraving tool 33 and has a gradually increasing diameter upward in the vertical direction Z. The enlarged diameter part 33a may have a diameter that increases continuously (see Fig. 14) or increases in steps. Examples of the engraving tool 33 include an end mill, a drill, and a fraise.

The liner portion forming unit 50B may have a removal unit (not shown) for removing a part of the engraved surface 10a of the base part 10. Examples of the removal unit include a blower to send air for blowing a part of the engraved portion and an aspirator to aspirate a part of the engraved portion.

In the liner portion forming step in the second embodiment, while a part of the engraving tool 33 is inserted into the base part 10, the relative position of the engraving tool 33 in a planar direction to the base part 10 is changed to form a liner portion 20B. When the relative position is changed, at least one of the engraving tool 33 and an object 70 is relatively transferred to the other such that the relative position moves along a path 24.

In the second embodiment, while the insertion depth K of the enlarged diameter part is changed according to a pixel predetermined value extracted in the extraction step, a liner portion 20B is formed.

For example, when an intended image is binarized in the color tone conversion step, the predetermined value is expressed in two gradations of 0 and 255. According to the predetermined value, the depth K may be controlled in two levels. The depth K may be set to small when the predetermined value is 0, and the depth K may be set to large when the predetermined value is 1, or vice versa. When the depth K is small, a liner portion 20B has a relatively small width W, and when the depth K is large, a liner portion 20B has a relatively large width W. When the depth K is small, a portion of the liner portion 20B corresponding to a narrow portion 20b is formed. When the depth K is large, a portion of the liner portion 20B corresponding to a wide portion 20a is formed.

When an intended image is gray-scaled in the color tone conversion step, the predetermined value is expressed in 256 gradations from 0 to 255. For example, the predetermined values may be divided into three levels of 0 to 100, 101 to 200, and 201 to 255, and the depth K may be controlled in three levels according to the predetermined value. For example, when the predetermined value is 0 to 100, the depth K may be set to a minimum value; when the predetermined value is 201 to 255, the depth K may be set to a maximum value; and when the predetermined value is 101 to 200, the depth may be set to a value between the depth K when the predetermined value is 0 to 100 and the depth K when the predetermined value is 201 to 255. The predetermined values may be divided into any levels. The predetermined values may be divided into two levels or into 256 levels. The threshold levels for dividing the predetermined values into a plurality of levels are not limited.

In accordance with the image tone conversion system, the gradations of a 256-gradation image may be limited to two gradations, four gradations, eight gradations, 16 gradations, or the like to set the speed. For example. when an image is converted into two gradations, the relative transfer speed V may be set to low speed for a portion having a predetermined value of 0 to 127, and the relative transfer speed V may be set to high speed for a portion having a predetermined value of 128 to 255. In a similar manner, when an image is converted into four gradations, predetermined values may be divided into, for example, 0 to 63, 64 to 127, 128 to 191, and 192 to 255, and the speed may be changed in four levels from low to slow. The same is applied to cases of eight gradations, 16 gradations, and the like.

The depth K may be changed continuously or be changed in steps.

By the production method in the second embodiment, as with the production method in the first embodiment, a liner portion 20 creating a pattern using subjective contours can be efficiently produced, and this enables efficient production of a cosmetic 1 having a three-dimensional pattern which is capable to express shades. By the production method in the second embodiment, a liner portion 20B as a groove can be easily formed.

The depth K in the perpendicular direction is not specifically limited, but is preferably 0.05 mm or more, more preferably 0.1 mm or more, and even more preferably 0.15 mm or more from the viewpoint of forming a three-dimensional pattern.

The depth K in the perpendicular direction is preferably 1 mm or less, more preferably 0.5 mm or less, and even more preferably 0.3 mm or less from the viewpoint of preventing an object 70 between the adjacent liner portions 20B from collapsing.

The solid personal care product of the present invention includes products applicable to the human body by applying the product onto the skin or by applying, spraying, or dropping, onto the skin, a liquid in which the product is dissolved or dispersed in a solvent such as water. The product may be applied with an applicator such as a puff and a brush or may be directly applied without any applicator.

Such a solid personal care product includes, for example, one or two or more selected from a cosmetic, a cleaning agent, and a bathing agent. The cosmetic includes a makeup cosmetic, a skin care cosmetic, a hair care product, and the like. The cleaning agent includes a solid shampoo, a soap paste, a solid soap, and the like. The makeup cosmetic includes an eye shadow and a foundation containing cosmetic powder and the like and a solid cosmetic such as a lipstick containing an oil agent and the like.

The solid personal care product is preferably solid at 1 atmosphere and 20°C from the viewpoint of enabling the formation of a three-dimensional shape.

The solid personal care product of the present invention includes a product that stimulates the senses of touch, smell, taste, and the like to maintain or improve health. For example, the product may be a massage tool such as a massage plate or a massage rod, a stainless soap, a solid deodorant such as activated carbon, or a solid air freshener such as an aroma candle or an aroma block.

The composition M constituting the liner portion 20 will next be described.

Unless otherwise noted, the states (three states) of a substance described below are based on 1 atmosphere and 20°C.

The composition M may contain a single ingredient or a plurality of ingredients.

The composition M preferably has a viscosity of 0.1 Pa·s or more, more preferably 0.5 Pa·s or more, and even more preferably 1 Pa·s or more from the viewpoint of improving the uniform dispersibility of a material contained in the composition M to stabilize the quality and of preventing a deposit discharged from a nozzle or a liner portion 20 from collapsing to stabilized the shape.

The viscosity is preferably 1 000 Pa·s or less, more preferably 500 Pa·s or less, and even more preferably 200 Pa·s or less from the viewpoint of improving the dischargeability to improve the formability.

To determine the viscosity of a composition M, the temperature of the composition M is set equal to the temperature of the composition M supplied from a nozzle or is set to the temperature during supplying. For example, when a composition M is a slurry or a substance other than a heated melt and is supplied from a nozzle at room temperature (25°C), the viscosity is determined at 25°C by using a Brookfield viscometer (manufactured by Toki Sangyo Co., Ltd, a digital viscometer, TVB-10R). In this case, the measurement conditions are as follows: any of rotors Nos. M1, M2, M3, M4, H1, H2, H3, H4, H5, H6, and H7 is used as the rotor according to the viscosity range of a sample; the rotation rate is set at 0.5 to 100 rpm; and an auto-stop function is used for the measurement time. When a composition M is a heated melt, the temperature of the composition M is set equal to the temperature of the composition supplied from a nozzle 61, and the viscosity is determined in the above measurement conditions.

The composition M preferably contains one or two or more selected from solids such as a powder and oil agents.

As such a solid, for example, one or two or more selected from powders used as a typical cosmetic ingredient, such as a color pigment, an extender pigment, a bright pigment, and an organic powder, is preferably contained.

As the color pigment, for example, one or two or more selected from metal oxides such as titanium oxide, zinc oxide, yellow iron oxide, colcothar, black iron oxide, iron blue, ultramarine, chromium oxide, and chromium hydroxide; metal complexes such as manganese violet and cobalt titanate; inorganic pigments such as carbon black; synthetic organic pigments such as Red 3, Red 104, Red 106, Red 201, Red 202, Red 204, Red 205, Red 220, Red 226, Red 227, Red 228, Red 230, Red 401, Red 405, Red 505, Orange 203, Orange 204, Orange 205, Yellow 4, Yellow 5, Yellow 401, Blue 1, and Blue 404; and natural organic pigments such as β-carotene, caramel, and paprika pigment may be contained.

As the extender pigment, for example, one or two or more selected from inorganic pigments such as silicic acid, silicic anhydride, magnesium silicate, talc, sericite, boron nitride, mica, synthetic mica, glass flake, synthetic phlogopite, kaolin, clay, bentonite, bismuth oxychloride, zirconium oxide, magnesium oxide, aluminum oxide, calcium sulfate, barium sulfate, magnesium sulfate, calcium carbonate, magnesium carbonate, aluminum silicate, magnesium silicate, silica, and alumina and composite powders thereof may be contained.

As the bright pigment, one or two or more selected from pigments prepared by coating the surface of plate-like particles of mica, synthetic phlogopite, glass, silica, alumina, or the like with a colorant such as titanium oxide, iron oxide, silicon oxide, iron blue, chromium oxide, tin oxide, chromium hydroxide, gold, silver, carmine, and an organic pigment; and pigments prepared by cutting a raw film of a polyethylene terephthalate-polymethyl methacrylate laminated powder, a polyethylene terephthalate-aluminum deposited powder, and a polyethylene terephthalate-gold deposit laminated powder may be contained.

As the organic powder, one or two or more selected from a silicone rubber powder, a silicone resin-coated silicone rubber powder, polymethylsilsesquioxane, a polyamide powder, a nylon powder, a polyester powder, a polypropylene powder, a polystyrene powder, a polyurethane powder, a vinyl resin powder, a urea resin powder, a phenol resin powder, a fluorine resin powder, a silicone resin powder, an acrylic resin powder, a melamine resin powder, a polycarbonate resin, a divinylbenzene-styrene copolymer, a silk powder, a wool powder, a cellulose powder, a long-chain alkyl phosphate metal salt, an N-mono-long-chain alkylacyl basic amino acid, and composites thereof may be contained.

These color pigments, extender pigments, and bright pigments are colored or non-colored (for example, white or substantially transparent) and can provide at least one effect of coloring, light diffraction, oil absorption, translucency, opacity, glossiness, a mat appearance, smoothness, and the like for a base or the skin .

The content of the powder in the composition M containing a solvent varies with the purpose thereof, but is preferably 10% by mass or more, more preferably 15% by mass or more, and even more preferably 20% by mass or more from the viewpoint of productivity such as preventing drying.

The content of the powder in the composition M containing a solvent is preferably 85% by mass or less, more preferably 80% by mass or less, and even more preferably 70% by mass or less from the viewpoint of productivity such as maintaining the flowability during supply.

Within such a range, a solid personal care product having a high-definition three-dimensional shape can be easily produced, and the feeling of use can be improved when the product is used.

The average particle size of the powder in the composition M is preferably 300 µm or less, more preferably 150 µm or less, and even more preferably 100 µm or less from the viewpoint of preventing the nozzle from clogging to enable stable discharge continuously.

The average particle size is a cumulative volume particle size D50 at a cumulative volume of 50% by volume determined with a laser diffraction/scattering particle size distribution analyzer. To determine the average particle size of a powder from a final product, first, the product is dissolved in water or oil to dissolve binder components, and particles are dispersed in the solvent. A particle size analyzer is then used to measure the particle size distribution of the solid, and the cumulative volume particle size D50 of the measurement result is determined to be the average particle size.

The average particle size of the powder in the composition M is preferably smaller than the cross sectional length D1 of the nozzle from the viewpoint of the supply stability from the nozzle. For a nozzle having a cross section that is an imperfect circle, the minimum length of the cross section of the nozzle is determined to be D1. The ratio of the average particle size of the powder in the composition to the cross sectional length D1 of the nozzle 61 (average particle size/nozzle cross sectional length) is preferably less than 1, more preferably 0.5 or less, even more preferably 0.35 or less, and further preferably 0.3 or less from the viewpoint of reducing the line width W1 of a deposit produced by the above method or a liner portion 20 in a plan view and of stably forming a high-definition three-dimensional design.

The ratio of average particle size/nozzle cross sectional length is preferably smaller but is practically 0.001 or more.

As the oil agent containable in the composition M, one or two or more selected from oils that are liquid at 1 atmosphere and 20°C (hereinafter also called liquid oils) and oils that are solid at 1 atmosphere and 20°C (hereinafter also called solid oils ) is preferably contained.

Examples of the liquid oil include a straight-chain or branched hydrocarbon oil, a vegetable oil, an animal oil, an ester oil, a silicone oil, and a polymer alcohol.

Examples of the straight-chain or branched hydrocarbon oil include liquid paraffin and squalane. As the vegetable oil, one or two or more selected from jojoba oil, olive oil, and the like is preferably contained.

Examples of the animal oil include liquid lanolin. Examples of the ester oil include a monohydric alcohol fatty acid ester and a polyhydric alcohol fatty acid ester. As the silicone oil, one or two or more selected from dimethylpolysiloxane, dimethylcyclopolysiloxane, methylphenylpolysiloxane, methylhydrogenpolysiloxane, a higher alcohol-modified organopolysiloxane, and the like is preferably contained.

As the polymer alcohol, one or two or more selected from polyethylene glycol and the like is preferably contained.

As the solid oil, one or two or more selected from vaseline, cetanol, stearyl alcohol, ceramide, and the like is preferably contained.

The composition M preferably further contain a liquid medium. The liquid medium is a liquid that can be used as a solvent or a dispersion medium for dissolving or dispersing a cosmetic. When a composition M in the slurry form is used, the composition M is preferably a mixture containing at least a powder and a liquid medium. When a composition M in the cosmetic slurry form is used, the composition M is preferably a mixture containing at least a powder containing the above pigment, an oil agent, and a liquid medium.

As the above liquid (liquid medium), for example, a substance having volatility in the liquid state (volatile solvent) is preferably contained.

Specifically, as the liquid (liquid medium), one or two or more selected from water, alcohols, ketones, hydrocarbons, and the like is preferably contained.

As the alcohol, one or two or more selected from monovalent chain aliphatic alcohols having 1 to 6 carbon atoms, monovalent cyclic aliphatic alcohols having 3 to 6 carbon atoms, monovalent aromatic alcohols, and the like is preferably contained.

As the specific example thereof, one or two or more selected from ethanol, isopropyl alcohol, butyl alcohol, phenylethyl alcohol, propanol, pentanol, and the like is preferably contained.

As the ketone, one or two or more selected from chain aliphatic ketones having 3 to 6 carbon atoms, cyclic aliphatic ketones having 3 to 6 carbon atoms, aromatic ketones having 8 to 10 carbon atoms, and the like is preferably contained.

As the specific example thereof, one or two or more selected from acetone, methyl ethyl ketone, methyl isobutyl ketone, cyclohexanone, acetophenone, and the like is preferably contained.

As the hydrocarbon, one or two or more selected from isoparaffin hydrocarbons and the like is preferably contained.

As the specific example thereof, IP solvent is preferably contained.

For a composition M containing a liquid medium, the content of the liquid medium in the composition M varies with the purpose thereof, but is preferably 15% by mass or more, more preferably 20% by mass or more, and even more preferably 30% by mass or more in terms of the total amount.

The content of the liquid medium in the composition M is preferably 90% by mass or less, more preferably 85% by mass or less, and even more preferably 80% by mass or less in terms of the total amount.

Within such a range, the composition M can have higher handling properties while the uniform dispersibility of constituent materials therein is improved.

The base part 10 and the liner portion 20 preferably contain one or two or more selected from solids such as a powder and oil agents. As the solid such as a powder and the oil agent, similar solids and oil agents to those contained in the composition M may be used.

The average particle size of the powder in the base part 10 and the liner portion 20 is preferably 0.1 µm or more and more preferably 1 µm or more from the viewpoint of adjusting tinting strength or optical properties such as brightness and saturation.

The content of the oil agent in the base part 10 and the liner portion 20 varies with the purpose thereof, but is preferably 0.5% by mass or more, more preferably 1% by mass or more, and even more preferably 1.5% by mass or more in terms of the total amount.

The content of the oil agent in the base part 10 and the liner portion 20 is preferably 30% by mass or less, more preferably 20% by mass or less, and even more preferably 15% by mass or less.

Within such a range, the solid personal care product can have higher color developability or higher feeling.

According to the type of an intended solid personal care product, the base part 10 and the liner portion 20 may appropriately contain one or two or more ingredient selected from a thickener, a film forming agent, a surfactant, a sugar, a polyhydric alcohol, a watersoluble polymer, a sequestrant, a lower alcohol, an amino acid, an organic amine, a pH adjuster, a skin conditioning agent, a vitamin, an antioxidant, a fragrance, a preservative, an ultraviolet absorber, an ultraviolet scattering agent, and the like, as long as the effect of the invention is not impaired.

As the ultraviolet absorber, one or two or more selected from benzophenone derivatives, methoxycinnamic acid derivatives, and the like is preferably contained.

As the benzophenone derivative, one or two or more selected from dihydroxybenzophenone, dihydroxydimethoxybenzophenone, hydroxymethoxybenzophenone sulfonate, and dihydroxydimethoxybenzophenone disulfonate is preferably contained.

As the methoxycinnamic acid derivative, one or two or more selected from 2-ethylhexyl methoxycinnamate and the like is preferably contained. As the ultraviolet scattering agent, for example, microparticles having an average particle size of 0.1 µm or less are preferably contained.

As the ultraviolet scattering agent, one or two or more selected from zinc oxide, titanium oxide, silica, and the like is preferably contained.

The base part 10 and the liner portion 20 preferably contain a powder as the solid content thereof at preferably 70% by mass or more, more preferably 80% by mass or more, and even more preferably 85% by mass or more from the viewpoint of producing a solid personal care product mainly containing the powder, such as a powder cosmetic.

The base part 10 and the liner portion 20 preferably contain a powder as the solid content thereof at preferably 99% by mass or less from the viewpoint of formability.

To make the liner portion 20 have such a formulation, for example, a powder is added to a composition M within the above solid content range. Alternatively, a composition M containing a liquid medium and a powder is supplied onto an object, and then the solidification step is performed to remove the liquid medium. Through such an operation, the formulation can be achieved.

The present invention has been described on the basis of the embodiments and aspects thereof, but the present invention can be appropriately modified without limitation by the embodiments and the aspects.

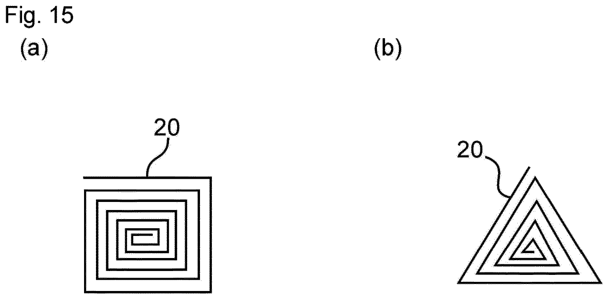

For example, in the cosmetic 1 shown in Fig. 1, the planar shape of the liner portion 20 is circular spiral. Alternatively, the planar shape may be quadrangular spiral (see Fig. 15(a)), triangular spiral (see Fig. 15(b)), or the like. In Figs. 15(a) and (b), no sparse-dense structure of the liner portion 20 is shown for convenience of explanation.

In the cosmetic 1 shown in Fig. 1, the surface 10a of the base part 10 is a flat face. Alternatively, the surface may be a curved face.

For example, with the liner portion forming unit 50B shown in Fig. 14, a liner portion 20B as a groove is formed by the engraving tool 33. Alternatively, a solid personal care product having a liner portion 20B as a groove may be formed by using an additive manufacturing machine, or what is called a 3D printer. A liner portion 20 as a ridge may be formed by using an additive manufacturing machine. A liner portion 20 may be directly formed on the surface 10a of a base part 10 by using an additive manufacturing machine, or a liner portion 20 formed by using an additive manufacturing machine may be placed on the surface 10a of a base part 10. A solid personal care product having a liner portion 20 as a ridge may be formed by using an additive manufacturing machine.

A liner portion 20 may be formed by press-molding an object 70. For the press molding, a liner portion 20 is formed by using a mold having a design corresponding to the liner portion 20 as the mold for press molding.

By the method for producing a solid personal care product of the present invention, a solid personal care product may be produced on demand. Specifically, the method for producing a solid personal care product on demand preferably includes a receiving step of receiving an image intended by an image provider such as a general consumer, a designer, and a creator through an image transmission means such as a network and a memory medium. The network is typically the Internet.

In the method for producing a solid personal care product on demand, a solid personal care product is preferably produced by the method for producing a solid personal care product in the first or second embodiment, on the basis of the image received in the receiving step.

More specifically, a solid personal care product is preferably produced on the basis of the received image, through at least the extraction step and the liner portion forming step, such that the pattern using subjective contours is formed on the surface 10a of a base part 10. The subjective contour is preferably formed as follows: the occupied area ratio S1 of the liner portion 20, 20B per unit area S0 in a plan view or an oblique downward view of the surface 10a changes along at least one direction parallel to the surface 10a; and the subjective contour is formed due to the change of the occupied area ratio S1/S0 forms. In the liner portion forming step, a composition M to constitute the liner portion may be discharged from a nozzle 61 to the base part 10 and be deposited on the base part, or a part of the surface 10a of the base part 10 may be engraved, and accordingly, the liner portion may also be formed.

In the receiving step, an intended image by an image provider may be received through an image transmission medium such as a memory medium. The image transmission medium may be a photograph, a painting, or the like. For example, an intended image may be received by importing a photograph through a scanner or the like.

### Examples

The present invention will next be described in further detail with reference to examples. However, the scope of the invention is not limited to the examples.

### [Example 1]

In a similar manner to the production method in the first embodiment, a solid personal care product in Example 1 was produced. As the intended image 40, the image shown in Fig. 12(b) was used. The used image was expressed in grayscale or in 256 gradations. Pixel predetermined values extracted in the extraction step were luminance expressed in 256 gradations from 0 to 255. In the liner portion forming step, the relative transfer speed V was set at a value calculated in accordance with V = (600/255)X + 200, and the distance F between the base part 10 and the nozzle 61 was set at a value calculated in accordance with F = (-0.2/255)X + 0.4. X was the above predetermined value. When the predetermined value was 0, the relative transfer speed V was set at 200 mm/min, and the distance F between the base part 10 and the nozzle 61 was set at 0.4 mm. When the predetermined value was 255, the relative transfer speed V was set at 800 mm/min, and the distance F between the base part 10 and the nozzle 61 was set at 0.2 mm. In the liner portion forming step, the supply speed Q of the composition M supplied from the nozzle 61 toward the surface 10a of the base part 10 was set at 0.74 mm³/s. A foundation formed in this manner was regarded as Example 1. The solid personal care product in Example 1 is shown in Table 1.

The formulations of the composition M and the base part 10 are shown below.

### <Formulation of composition M>

(1) Powder ingredient
   · Pearl powder: 52 parts by mass
   · Pigment powder: 38.95 parts by mass
(2) Ingredients other than powder
   - Water: 90 parts by mass
   - Oil agent: 3 parts by mass
   - Wetting agent: 2 parts by mass
   - Thickener: 0.55 parts by mass
   - Preservative and other ingredients: 3.5 parts by mass

### <Formulation of base part 10>

(1) Powder ingredient
   - Pigment powder: 89 parts by mass
(2) Ingredients other than powder
   - Oil agent: 11 parts by mass

**[Table 1]**

| | Example 1 | Comparative Example 1 |
|---|---|---|
| Product | Foundation | Foundation |
| Image processing in color tone conversion step | Gray scaling | - |
| Pixel predetermined values extracted in extraction step | Luminance of 256 gradations | - |
| Liner portion formation method in liner portion forming step | A composition is discharged from the nozzle and is deposited on the surface of the base part, and a liner portion is formed. | - |
| What are changed to form sparse-dense structure | The width and height of the liner portion | - |
| What are changed to change occupied area ratio S1/S0 in liner portion forming step | The relative transfer speed of the nozzle to the object calculated in accordance with V = (600/255)X + 200, | - |
| | The distance from the nozzle calculated in accordance with F = (-0.2/255)X + 0.4, | |
| | Composition supply speed: Q = 0.74 mm³/s | |
| Product photograph | 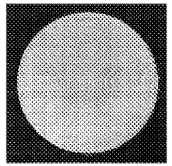 | 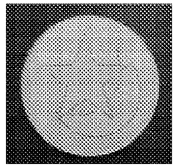 |
| Width W1 of widest portion of liner portion (mm) | 0.91 | 0.57 |
| Width W2 of narrowest portion of liner portion (mm) | 0.32 | 0.30 |
| Ratio of width W2 to width W1 (W2/W1) (%) | 35.16 | 52.63 |
| Height T1 of highest portion of liner portion (mm) | 0.33 | 0.26 |
| Height T2 of lowest portion of liner portion (mm) | 0.17 | 0.23 |
| Ratio of height T2 to height T1 (T2/T1) (%) | 51.52 | 88.46 |
| Longest distance D1 between liner portions (mm) | 0.76 | - |
| Shortest distance D2 between liner portions (mm) | 0.33 | - |
| Ratio of distance D1 to width W1 (D1/W1) (%) | 83.52 | - |
| Ratio of distance D2 to width W2 (D2/W2) (%) | 103.13 | - |
| Ratio of distance D2 to distance D1 (D2/D1) (%) | 43.42 | - |

### [Comparative Example 1]

A composition was discharged from the nozzle to the surface of a base part and was deposited on the surface, and a pattern of the deposit was formed on the surface of the base part. Accordingly, a solid personal care product in which a pattern was formed on the surface of the base part was produced.

When the composition was discharged from the nozzle, the relative transfer speed of the nozzle to the base part was set at 40 mm/s; the distance between the base part and the nozzle was set at 0.3 mm; and the supply speed of the composition supplied from the nozzle toward the surface of the base part was set at 6 mm³/s. A foundation formed as above was regarded as Comparative Example 1. The foundation in Comparative Example 1 is shown in Table 1.

The formulation of the composition was the same as the formulation of the composition M in Example 1, and the formulation of the base part was the same as the formulation of the base part 10 in Example 1.

In Example 1, a solid personal care product having a three-dimensional pattern which is capable to express shades was produced as shown in Table 1. In contrast, in the foundation in Comparative Example 1, the pattern of the foundation failed to express shades as shown in Table 1.

This reveals that the present invention enables the production of a solid personal care product having a three-dimensional pattern which is capable to express shades.

### [Example 2]

In a similar manner to the production method in the first embodiment, a solid personal care product in Example 2 was produced. As the intended image 40, the image shown in Fig. 12(c) was used. The used image was expressed in grayscale or in 256 gradations. In the color tone conversion step, luminance values of 0 to 255 were converted into 8 gradations, and the resulting values were used. Pixel predetermined values extracted in the extraction step were luminance expressed in 256 gradations from 0 to 255. The above values converted into 8 gradations were used as the luminance. In the liner portion forming step, the relative transfer speed V was set at a value calculated in accordance with V = (1500/255)X + 300, and the distance F between the base part 10 and the nozzle 61 was set at a value calculated in accordance with F = (-0.2/255)X + 0.4. X was the above predetermined value. When the predetermined value was 0, the relative transfer speed V was set at 300 mm/min, and the distance F between the base part 10 and the nozzle 61 was set at 0.4 mm. When the predetermined value was 255, the relative transfer speed V was set at 1 800 mm/min, and the distance F between the base part 10 and the nozzle 61 was set at 0.2 mm. In the liner portion forming step, the supply speed Q of the composition M supplied from the nozzle 61 toward the surface 10a of the base part 10 was set at 1.86 mm³/s. An aroma candle produced as above was regarded as Example 2. The solid personal care product in Example 2 is shown in Table 2.

The formulations of the composition M and the base part 10 are shown below.

### <Formulation of composition M>

### Marabu candle liner (Brown)

The candle liner described in https://www.kameyama-candle.jp/ec/shop/category_items_detail.htm?Item=X9890204 was used.

### <Formulation of base part 10>

As the base part 10, Merry berries & Mimosa Candle (Single Wick, 190 g) manufactured by Molton Brown was used.

**[Table 2]**

| | Example 2 |
|---|---|
| Product | Aroma candle |
| Image processing in color tone conversion step | Gray scaling (converted into 8 gradations) |
| Pixel predetermined values extracted in extraction step | Luminance of 256 gradations |
| Liner portion formation method in liner portion forming step | A composition is discharged from the nozzle and is deposited on the surface of the base part, and a liner portion is formed. |
| What are changed to form sparse-dense structure | The width and height of the liner portion |
| What are changed to change occupied area ratio S1/S0 in liner portion forming step | The relative transfer speed of the nozzle to the object calculated in accordance with V = (1500/255)X - 300, |
| | The distance from the nozzle calculated in accordance with F = -(0.2/255)X + 0.4, Composition supply speed: Q = 1.86 mm³/s |
| Product photograph | 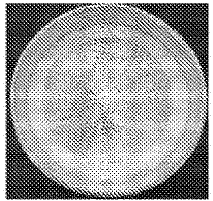 |
| Width W1 of widest portion of liner portion (mm) | 1.08 |
| Width W2 of narrowest portion of liner portion (mm) | 0.34 |
| Ratio of width W2 to width W1 (W2/W1) (%) | 31.48 |
| Height T1 of highest portion of liner portion (mm) | 0.32 |
| Height T2 of lowest portion of liner portion (mm) | 0.16 |
| Ratio of height T2 to height T1 (T2/T1) (%) | 50.00 |
| Longest distance D1 between liner portions (mm) | 0.96 |
| Shortest distance D2 between liner portions (mm) | 0.36 |
| Ratio of distance D1 to width W1 (D1/W1) (%) | 88.89 |
| Ratio of distance D2 to width W2 (D2/W2) (%) | 105.88 |
| Ratio of distance D2 to distance D1 (D2/D1) (%) | 37.50 |

In Example 2, a solid personal care product having a three-dimensional pattern which is capable to express shades was produced as shown in Table 2.

### Industrial Applicability

According to the present invention, a solid personal care product having a three-dimensional pattern capable expressing shades can be provided.

## Claims

1. A solid personal care product comprising:
a base part; and
a liner portion formed on a surface of the base part, wherein
in a plan view or an oblique downward view of the surface, a change of a ratio of an occupied area by the liner portion per unit area is made along at least one direction parallel to the surface, and
the change of the ratio of the occupied area is configured to show a pattern using a subjective contour.

2. The solid personal care product according to claim 1, wherein in a plan view or an oblique downward view of the surface, a sparse-dense structure including a dense portion in which the liner portion is densely located and a sparse portion in which the liner portion is sparsely located is formed, and
the sparse-dense structure is formed to give the change of the ratio of the occupied area.

3. The solid personal care product according to claim 1 or 2, wherein the liner portion is arranged not to intersect a part of the liner portion or another liner portion.

4. The solid personal care product according to any one of claims 1 to 3, wherein the number of the liner portion formed on the solid personal care product is one.

5. The solid personal care product according to any one of claims 1 to 4, wherein a change of any one of or both of a width and a height of the liner portion along the one direction is made to give the change of the ratio of the occupied area.

6. The solid personal care product according to any one of claims 1 to 5, wherein a curve, a bend, or a curve and a bend of the liner portion are made to give the change of the ratio of the occupied area.

7. The solid personal care product according to any one of claims 1 to 6, wherein a part including the base part and the liner portion has a laminate structure in which two or more layers are laminated.

8. The solid personal care product according to claim 7, wherein compositions constituting the layers in the laminate structure differ from each other in any one or both of formulation and color.

9. The solid personal care product according to claim 7 or 8, wherein the liner portion is formed of a layer most distant from a layer constituting the base part in a part having the laminate structure.

10. The solid personal care product according to any one of claims 1 to 9, wherein at least one of a hue contrast, a lightness contrast, and a saturation contrast of the pattern is configured to be changed depending on a viewing direction.

11. The solid personal care product according to any one of claims 1 to 10, wherein an arrangement pattern of the liner portion in a plan view is a stripe pattern.

12. The solid personal care product according to any one of claims 1 to 11, wherein in a plan view, the liner portion has a width of 0.05 mm or more and 3 mm or less, preferably 0.1 mm or more and 2 mm or less, and more preferably 0.15 mm or more and 1.5 mm or less.

13. The solid personal care product according to any one of claims 1 to 12, wherein in a plan view, a ratio of a width of the narrowest portion of the liner portion to a width of the widest portion of the liner portion is 1.6% or more and 90% or less, preferably 2.5% or more and 80% or less, and more preferably 3.3% or more and 70% or less.

14. The solid personal care product according to any one of claims 1 to 13, wherein the liner portion is a ridge formed on the surface of the base part, and the liner portion has a height of 0.05 mm or more and 2 mm or less, preferably 0.1 mm or more and 1 mm or less, and more preferably 0.12 mm or more and 0.6 mm or less, or
the liner portion is a groove formed on the surface of the base part, and the liner portion has a depth of 0.05 mm or more and 2 mm or less, preferably 0.1 mm or more and 1 mm or less, and more preferably 0.12 mm or more and 0.6 mm or less.

15. The solid personal care product according to any one of claims 1 to 14, wherein the liner portion is a ridge formed on the surface of the base part, and a ratio of a height of the lowest portion of the liner portion to a height of the highest portion of the liner portion is 2.5% or more and 90% or less, preferably 5% or more and 80% or less, and more preferably 8.3% or more and 70% or less, or
the liner portion is a groove formed on the surface of the base part, and a ratio of a depth of the shallowest portion of the liner portion to a depth of the deepest portion of the liner portion is 2.5% or more and 90% or less, preferably 5% or more and 80% or less, and more preferably 8.3% or more and 70% or less.

16. The solid personal care product according to any one of claims 1 to 15, wherein in a plan view, a distance between the adjacent liner portion(s) is 0.15 mm or more and 15 mm or less, preferably 0.5 mm or more and 10 mm or less, and more preferably 1 mm or more and 5 mm or less.

17. The solid personal care product according to any one of claims 1 to 16, wherein of the distance between the adjacent liner portions in a plan view, a ratio of the shortest distance D2 to the longest distance D1, D2/D1, is 5% or more and 100% or less, preferably 10% or more and 90% or less, and more preferably 20% or more and 80% or less.

18. The solid personal care product according to any one of claims 1 to 17, wherein in a plan view, a ratio of the distance between the adjacent liner portions to the width of the liner portion is 5% or more and 5 000% or less, preferably 7.5% or more and 4 000% or less, and more preferably 10% or more and 3 000% or less.

19. The solid personal care product according to any one of claims 1 to 18, wherein in a plan view, the liner portion has a curvature radius of 0.1 mm or more and 1 000 mm or less, preferably 0.2 mm or more and 800 mm or less, and more preferably 0.3 mm or more and 600 mm or less.

20. The solid personal care product according to any one of claims 1 to 19, wherein in a plan view, a ratio of a curvature radius at a position having the smallest curvature radius of the liner portion to a curvature radius at a position having the largest curvature radius is 0.01% or more and 90% or less, preferably 0.02% or more and 80% or less, and more preferably 0.03% or more and 70% or less.

21. A method for producing a solid personal care product having a pattern based on an intended image, wherein
the solid personal care product includes: a base part; and a liner portion formed on a surface of the base part,
in a plan view or an oblique downward view of the surface, a change of a ratio of an occupied area by the liner portion per unit area is made along at least one direction parallel to the surface, and
the change of the ratio of the occupied area is configured to show the pattern using a subjective contour, wherein
the method comprises:
extracting, from the intended image, measured values of pixels respective to multiple points on a formation scheduled position of the liner portion; and forming the liner portion on the surface of the base part, wherein,
when forming the liner portion, the measured values of the pixels extracted are referred to, and the ratio of the occupied area is changed by making the occupied area respective to measured values.

22. The method for producing a solid personal care product according to claim 21, wherein when forming the liner portion, a composition to constitute the liner portion is discharged from a nozzle toward the base part and is deposited on the base part to form the liner portion.

23. The method for producing a solid personal care product according to claim 22, wherein when forming the liner portion, the composition is discharged while at least one of a relative transfer speed of the nozzle to the base part, a distance between the base part and the nozzle, and a discharge amount of the composition from the nozzle is changed.

24. The method for producing a solid personal care product according to claim 22 or 23, wherein when forming the liner portion, the composition having flowability is discharged from the nozzle.

25. The method for producing a solid personal care product according to any one of claims 22 to 24, wherein when forming the liner portion, at least one of the nozzle and an object placement platform having the base part on the platform is transferred in a planar direction, a vertical direction, or a combination direction thereof to relatively transfer at least one of the nozzle and the base part on the object placement platform to the other.

26. The method for producing a solid personal care product according to claim 21, wherein when forming the liner portion, a part of the surface of the base part is engraved to form the liner portion.

27. The method for producing a solid personal care product according to claim 26, wherein when forming the liner portion, a relative position in a planar direction of an engraving tool to the base part is changed while a part of the engraving tool having an enlarged diameter part having a gradually increasing diameter upward in a vertical direction is inserted into the base part, and an insertion depth of the enlarged diameter part is changed referring to a pixel color value extracted to form the liner portion.

28. The method for producing a solid personal care product according to any one of claims 21 to 27, wherein the liner portion is drawable with a single stroke.

29. The method for producing a solid personal care product according to any one of claims 21 to 28, wherein the liner portion is drawn with a spiral pattern.

30. The method for producing a solid personal care product according to any one of claims 21 to 29, further comprising a converting of a color tone.

31. The method for producing a solid personal care product according to any one of claims 21 to 30, further comprising a setting of an interest region.

32. The method for producing a solid personal care product according to any one of claims 21 to 31, wherein the converting of the color tone and the setting of the interest region are performed before the extracting.

33. A method for producing a solid personal care product,
the method comprising:
receiving an intended image of an image provider through an image transmission medium such as a network or a memory medium; and
producing a solid personal care product based on the received image by the method for producing a solid personal care product according to any one of claims 21 to 32.
